# EUROPEAN PATENT SPECIFICATION

(11) **EP 0 476 983 B1**
(45) Date of publication and mention of the grant of the patent: **15.03.2000**
(21) Application number: 91308489.3
(22) Date of filing: 18.09.1991
(51) Int. Cl.: C07K 14/475, C07K 1/36, C12N 15/19, C12P 21/00, A61K 38/17

(54) **Vascular endothelial cell growth factor II**
Wachstumsfaktor II für vaskuläre Endothelzellen
Facteur II de croissance de cellules vasculaires endotheliales

(30) Priority: 21.09.1990 US 586638; 21.09.1990 US 586640
(43) Date of publication of application: 25.03.1992
(73) Proprietor: Merck & Co., Inc., Rahway New Jersey 07065-0900 (US)
(72) Inventor: Bayne, Marvin L., Westfield, NJ 07090 (US); Conn, Gregory L., Rahway, NJ 07065 (US); Thomas, Kenneth A., Jr., Chatham Burough, NJ 07928 (US)
(74) Representative: Thompson, John Dr.

(56) References cited:
- EP-A- 0 186 084
- EP-A- 0 399 816
- WORLD PATENS INDEX (Online), DERWENT PUBLICATIONS LTD., London Accession No. 90-181 364 & JP-A-2 117 698
- WORLD PATENTS INDEX (Online), DERWENT PUBLICATIONS LTD., London Accession No. 89-088 700 & JP-A-1 038 100
- Science 246 (1989) pages 1306-1309
- Science 246 (1989) pages 1309-1312

## Description

### BRIEF DESCRIPTION OF THE DRAWING

Figure 1. VEGF II activity present in fractions eluting from a polyaspartic acid WCX HPLC cation exchange column, bar denotes pooled active fractions.
Figure 2. VEGF II activity present in fractions eluting from a metal chelate column.
Figure 3. VEGF II activity present in fractions eluting from a RP-HPLC C₄ column.
Figure 4. Full length amino acid residue protein translation product and its cDNA coding sequence for VEGF I A subunit plus polypeptide cleavage products used to determine the amino acid sequence.
Figure 5. Full length amino acid residue protein translation product and its cDNA coding sequence for VEGF II A subunit plus polypeptide cleavage products used to determine the amino acid sequence. Refer to Example 9 *infra* for a discussion of the 164, 120 and 188 amino acid secreted forms of the VEGF II A subunit.
Figure 6. Full length amino acid residue protein translation product and its cDNA coding sequence for the 135 amino acid form of VEGF II B subunit plus polypeptide cleavage products used to determine the amino acid sequence.
Figure 7. Full length amino acid residue protein translation product and its cDNA coding sequence for the 115 amino acid form of VEGF II B subunit.

### BACKGROUND OF THE INVENTION

A new class of cell-derived dimeric mitogens with apparently restricted specificity for vascular endothelial cells has recently been identified and generally designated vascular endothelial growth factor (VEGF). The mitogen has been purified from: conditioned growth media of rat glioma cells, [Conn et al., Proc. Natl. Acad. Sci. USA 87: 2628-2632 (1990)]; conditioned growth media of bovine pituitary folliculo stellate cells [Ferrara and Henzel, Biochem. Biophys. Res. Comm. 161: 851-858 (1989) and Gospodarowicz et al., Proc. Natl. Acad. Sci. USA 86: 7311-7315 (1989)]. Vascular endothelial growth factor I (VEGF I) is a homodimer with an apparent molecular mass of 46 kDa, with each subunit having an apparent molecular mass of 23 kDa. VEGF I has distinct structural similarities to platelet-derived growth factor (PDGF), a mitogen for connective tissue cells but not vascular endothelial cells from large vessels.

### OBJECTS OF THE INVENTION

It is, accordingly, an object of the present invention to provide a novel vascular endothelial growth factor II (VEGF II) free of other proteins. Another object is to provide a procedure for the purification of the substantially pure VEGF II. A further object is to provide VEGF II to stimulate endothelial cells for induction of blood vessel growth, vascular repair and the production of artificial blood vessels. Another object is to provide VEGF II to stimulate tissue repair.

### SUMMARY OF THE INVENTION

Vascular endothelial cell growth factor II is purified from the culture media used to maintain mammalian glioma cells. The protein is a heterodimer and stimulates mitogenesis of mammalian vascular endothelial cells and is useful for the promotion of vascular development and repair. This unique growth factor is also useful in the promotion of tissue repair.

### DETAILED DESCRIPTION

The present invention relates to a unique vascular endothelial cell growth factor (designated VEGF II), isolated and purified from glioma cell conditioned medium, which exhibits mitogenic stimulation of vascular endothelial cells. Glioma is defined herein as any neoplasm derived from one of the various types of cells that form the interstitial tissue of the central nervous system including brain, spinal cord, posterior pituitary gland and retina. Consequently, the scope of the present invention is intended to include the unique growth factor isolated and purified from any mammalian glioma tissue or other cells including cell lines. Cell lines include, but are not limited to, glioma-derived cell lines such as C6, hs 683 and GS-9L; glioblastomas such as A-172 and T98G; neuroblastomas such as IMR-32 and SK-N-MC; neurogliomas such as H4; tetromas such as XB-2; astrocytomas such as U-87 MG and U-373 MG; embryonal carcinomas and non-transformed glial or astrocyte cell lines, and the human medulloblastoma line TE 671, with GS-9L and TE 671 being preferred. VEGF II is present and can be isolated from rat tissue including ovary, heart and kidney. Anterior pituitary tumor cell lines such as GH3 and Hs 199 may also be used. Although the VEGF of this invention is described as being isolated from rat cells, the same or substantially similar growth factor may be isolated from other mammalian cells, including human cells.

Vascular endothelial growth factor II may exist in various microheterogeneous forms which are isolated from one or more of the various cells or tissues described above. Microheterogeneous forms as used herein refer to a single gene product, that is a peptide produced from a single gene unit of DNA, which is structurally modified at the mRNA level or following translation. Peptide and protein are used interchangeably herein. The microheterogeneous forms will all have equivalent mitogenic activities. Biological activity and biologically active are used interchangeably and are herein defined as the ability of VEGF II to stimulate DNA synthesis in target cells including vascular endothelial cells as described below which results in cell proliferation. The modifications may take place either in vivo or during the isolation and purification process. In vivo modification results from, but is not limited to, proteolysis, glycosylation, phosphorylation, deamidation or acetylation at the N-terminus. Proteolysis may include exoproteolysis wherein one or more terminal amino acids are sequentially, enzymatically cleaved to produce microheterogeneous forms which have fewer amino acids than the original gene product. Proteolysis may also include endoproteolytic modification that results from the action of endoproteases which cleave the peptide at specific locations within the amino acid sequence. Similar modifications can occur during the purification process which also results in production of microheterogeneous forms. The most common modification occurring during purification is proteolysis which is generally held to a minimum by the use of protease inhibitors. Under most conditions one or more microheterogeneous forms are present following purification of native VEGF II. Native VEGF II refers to VEGF II isolated and purified from cells that produce VEGF II. Vascular endothelial growth factor II may also exist in various alternatively spliced forms which is defined herein as the production of related mRNAs by differential processing of exons. Exons are defined as those parts of the DNA sequence of a eukaryotic gene that code for the final protein product.

Glioma cells such as the rat cell line GS-9L are grown to confluence in tissue culture flasks, about 175 cm², in a cell culture medium such as Dulbecco's Modified Eagle's Medium (DMEM) supplemented with about 10% newborn calf serum (NCS). When the cells reach confluence the culture medium is removed, the cell layers are washed with Ca⁺⁺, Mg⁺⁺-free phosphate buffered saline (PBS) and are removed from the flasks by treatment with a solution of trypsin, about 0.1%, and EDTA, about 0.04%. The cells, about 1 x 10⁸, are pelleted by centrifugation, resuspended in about 1500 ml of DMEM containing about 5% NCS and plated into a ten level cell factory (NUNC), 6,000 cm² surface area. The cells are incubated for about 48 to about 96 hours, with 72 hours preferred, at about 37° C in an atmosphere of about 5% CO₂ . Following incubation the medium is removed and the cell factories are washed about 3 times with PBS. About 1500 ml of fresh culture media is added containing about a 1:2 mixture of Ham's-F12/DMEM containing about 15 mM Hepes, about 5 µg/ml insulin, about 10 µg/ml transferrin and with or without about 1.0 mg/ml bovine serum albumin. This media is replaced with fresh media after about 24 hr and collected every 48 hr thereafter. The collected conditioned media is filtered through Whatmen #1 paper to remove cell debris and stored at about -20°C.

The GS-9L conditioned medium is thawed and brought to pH 6.0 with 1 M HCl. The initial purification step consists of cation exchange chromatography using a variety of cation exchangers on a variety of matrices such as CM Sephadex C-50, Pharmacia Mono S, Zetachrom SP and Polyaspartic Acid WCX (Nest Group) with CM Sephadex C-50 (Pharmacia) being preferred. The VEGF-containing culture media is mixed with CM Sephadex C-50 at about 2 gm per about 20 L of the conditioned media and stirred at low speed for about 24 hr at 4° C. The resin is allowed to settle and the excess liquid is removed. The resin slurry is packed into a column and the remaining culture media is removed. Unbound protein is washed from the column with 0.05 M sodium phosphate, about pH 6.0, containing 0.15 M NaCl. The VEGF II is eluted with about 0.05 M sodium phosphate, about pH 6.0, containing about 0.6 M NaCl.

The active fractions collected from the CM Sephadex C-50 column are further fractionated by lectin affinity chromatography for additional purification of VEGF II. The lectins which may bind VEGF II include, but are not limited to, lectins which specifically bind mannose residues such as concanavalin A and lens culinaris agglutinin, lectins which bind N-acetylglucosamine such as wheat germ agglutinin, lectins that bind galactose or galactosamine and lectins which bind sialic acids, with concanavalin A (Con A) being preferred. A 0.9 cm diameter column containing about 5 ml packed volume of Con A agarose (Vector Laboratories) is washed and equilibrated with about 0.05 M sodium acetate about pH 6.0, containing about 1 mM CaCl₂, about 1 mM MnCl₂and about 0.6 M NaCl. The unbound protein is washed from the column with equilibration buffer. The VEGF II is eluted with about 0.1 M NaCl buffer containing about 0.32 M α-methyl mannoside and about 0.28 M α-methyl glucoside.

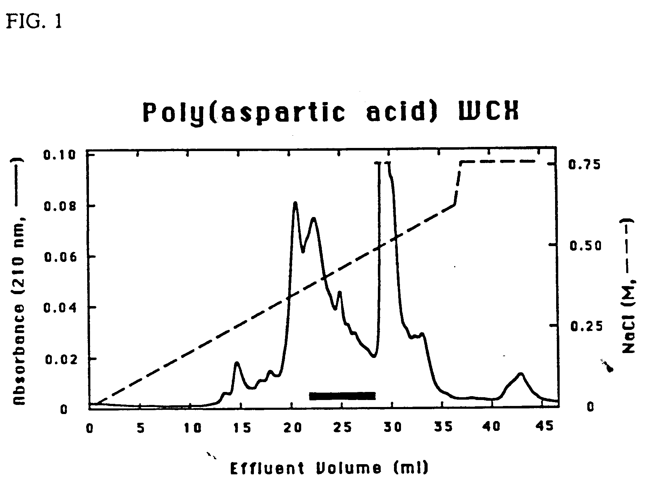

The VEGF II active eluate from the Con-A column is applied to a Polyaspartic Acid WCX cation exchange high performance liquid chromatography (HPLC) column, 4.6 mm x 250 mm, pre-equilibrated in about 0.05 M sodium phosphate buffer, pH 6.0. The column is eluted with a linear gradient of about 0 to 0.75 M NaCl in the phosphate buffer over about 60 minutes. The flow rate is maintained at about 0.75 ml/min collecting 0.75 ml fractions. Vascular endothelial growth factor activity is present in fractions eluting between approximately 21.7 and 28.5 ml see Fig. 1.

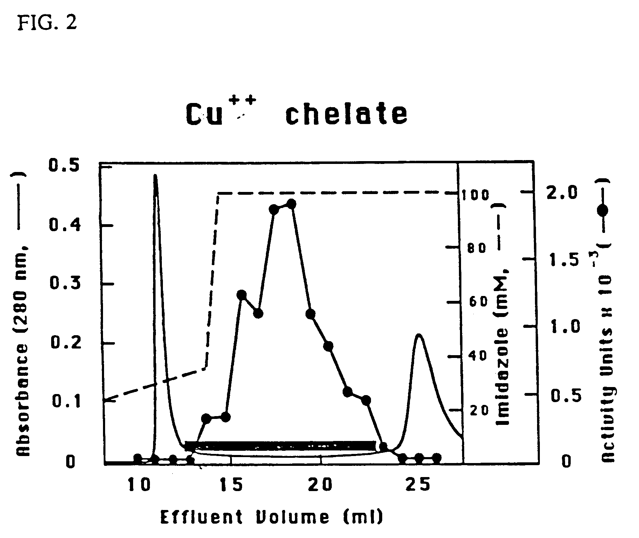

The active fractions eluted from the polyaspartic WCX column that contain VEGF II are pooled, adjusted to about pH 7.0 and loaded onto a 1 x 10 cm column of Pharmacia Chelating Sepharose 6B charged with an excess of copper chloride and equilibrated in about 0.05 M sodium phosphate, about pH 7.0, containing about 2 M NaCl and about 0.5 mM imidazole (A buffer). VEGF II is eluted from the column with a gradient from 0-20% B over 10 minutes, 20-35% B over 45 minutes and 35-100% B over 5 minutes at a flow rate of 0.3 ml/min, where B buffer is 0.05 M sodium phosphate, pH 7.0, containing about 2 M NaCl and 100 mM imidazole. The active fractions containing VEGF II activity eluted between about 12.6 and 22.8 ml of the gradient effluent volume, see Fig. 2.

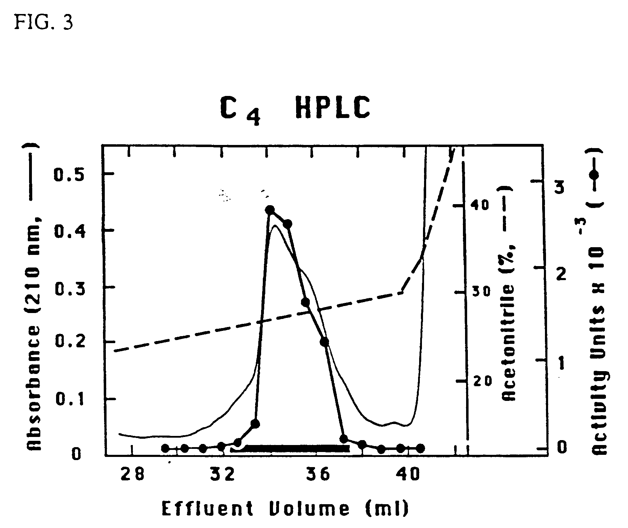

The pooled fractions containing VEGF II activity eluted from the metal chelate column are loaded onto a 4.6 mm x 5 cm Vydac C₄ reverse phase HPLC column (5 µm particle size) previously equilibrated in solvent A [0.1% trifluoroacetic acid (TFA)]. The column is eluted with a linear gradient of about 0 to 30% solvent B over 15 minutes, 30% B for an additional 15 minutes, then 30-45% B over 22.5 minutes and finally 45-100% B over 5.5 minutes. Solvent B consists of solvent A containing 67% acetonitrile (v/v). The flow rate is maintained at about 0.75 ml/min and fractions are collected every minute. The homogeneous VEGF II elutes from the C₄ column under these conditions at between about 32 and about 38 ml of the gradient effluent volume, see Fig. 3.

Purity of the protein is determined by sodium dodecylsulfate (SDS) polyacrylamide gel electrophoresis (PAGE) in 12.5% crosslinked gels using the technique of Laemmli, Nature 227: 680-684 (1970). The silver stained gels show VEGF II to consist of one band under non-reducing conditions with an approximate apparent molecular mass of about 58,000 daltons. When a sample containing the microheterogeneous forms of VEGF II is separated under reducing conditions it migrates as two about 23 kilodalton (kDa) subunits. The purification process results in VEGF II that is essentially free of other mammalian cell products, such as proteins. Recombinantly derived VEGF II will also be free of mammalian cell products.

Biological activity is determined by mitogenic assay using mammalian vascular endothelial cells. Human umbilical vein endothelial (HUVE) cells are plated on gelatin-coated dishes at a density of about 5000 cells per well in about 500 µl of Medium 199 (M199) containing about 20% heat-inactivated fetal calf serum (FCS). Samples to be assayed are added at the time of plating. The tissue culture plates are incubated at about 37° C for about 12 hours and about 2 microcuries of tritiated thymidine (NEN, 20 Ci/mmol) is added per ml of assay medium (1.0 µCi/well). The plates are incubated for a further 60 hr, the assay medium is removed and the plates are washed with Hanks balanced salt solution containing about 20 mM Hepes, about pH 7.5, and about 0.5 mg/ml bovine serum albumin. The cells are lysed and the labelled DNA solubilized with about 200 µl of a solution containing about 2 gm of sodium carbonate and about 400 mg sodium hydroxide in about 100 ml water. The incorporated radioactivity was determined by liquid scintillation counting.The concentration of VEGF which elicited a half-maximal mitogenic response in HUVE cells was approximately 2 ± 1 ng/ml. The glycosaminoglycan heparin, which is required in these assays at a level of 10-100 µg/ml to promote a response to a positive control, acidic fibroblast growth factor, does not enhance mitogenic stimulation of these cells by VEGF II.

A purified about 1-2 µg sample of VEGF II is reduced in about 0.1 M Tris, about pH 9.5, with about 0.1% EDTA, about 6 M guanidinium chloride and about 20 mM dithiothreitol for about 2 hr at about 50° C. The reduced protein is carboxymethylated for about 1 hourin a solution containing about 9.2 µM of unlabelled and 2.8 µM of ¹⁴C-iodoacetic acid in about 0.7 M Tris,about pH 7.8, and about 0. 1% EDTA and about 6 M guanidinium chloride. The protein is carboxymethylated for about 1 hr at room temperature. The protein is isolated after reduction and carboxymethylation by reverse phase HPLC chromatography on a Vydac C₄ column , about 4.6 mm x 5 cm. The protein is loaded onto a column pre-equilibrated with about 0.1% TFA and eluted by a 45 ml linear gradient from about 0.1% TFA to 0.1% TFA/67% acetonitrile at a flow rate of about 0.75 ml/min. The reduced and carboxymethylated protein eluted as two peaks at approximately 25 and 28 ml with the proportion being approximately equal as determined by monitoring absorbance at 210 nm.

Samples of the reduced and carboxymethylated monomers are applied to polybrene-coated glass fiber filters and their N-terminal sequences are determined by Edman degradation in an ABI gas phase microsequencer in conjunction with an ABI 120A on line phenylthiohydantoin analyzer following the manufacturers instructions. The protein showing the peak of absorbance eluting at approximately 28 ml (A subunit or monomer) yielded an amino terminal sequence of:
Ala Pro Thr Thr Glu Gly Glu Gln Lys Ala His Glu Val Val
which is identical to the monomers of VEGF I, Conn et al., Proc. Natl. Acad. Sci. USA 87: 2628-2632 (1990). The peak of absorbance eluting at approximately 25 ml (B subunit or monomer) yielded an N-terminal sequence of:
Ala Leu Ser Ala Gly Asn Xxx Ser Thr Glu Met Glu Val Val Pro Phe Asn Glu Val
plus a nearly equal amount of a truncated form of the same sequence missing the first three amino acid residues. The missing Xxx residue corresponds to an Asn residue in the cloned cDNA, see below. Since this missing Asn occurs in a classical Asn Xxx Ser/Thr N-glycosylation sequence it is presumed to be glycosylated. The A subunit and the total of both B subunits are recovered in nearly equal amounts supporting the interpretation that the two peptides combine to form an AB heterodimer in VEGF II.

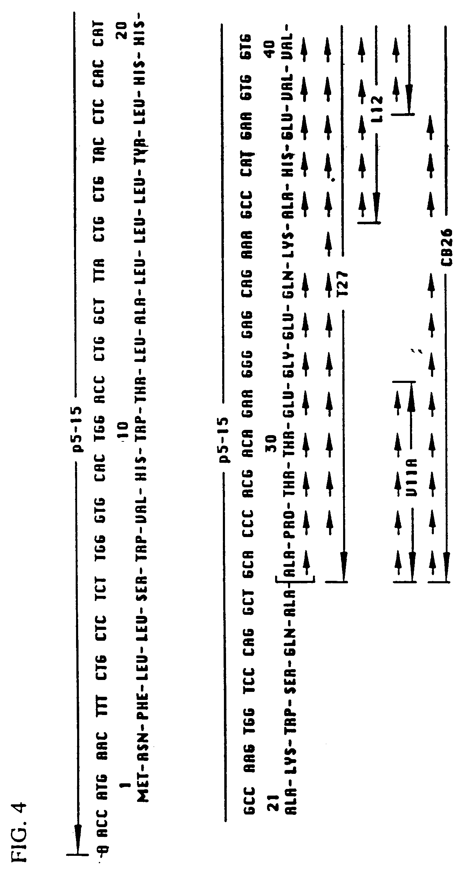

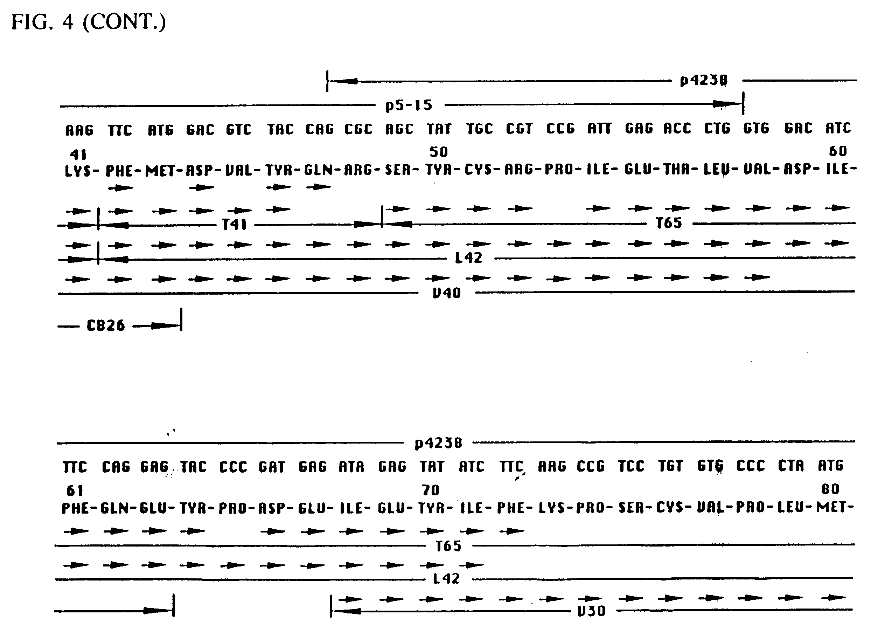

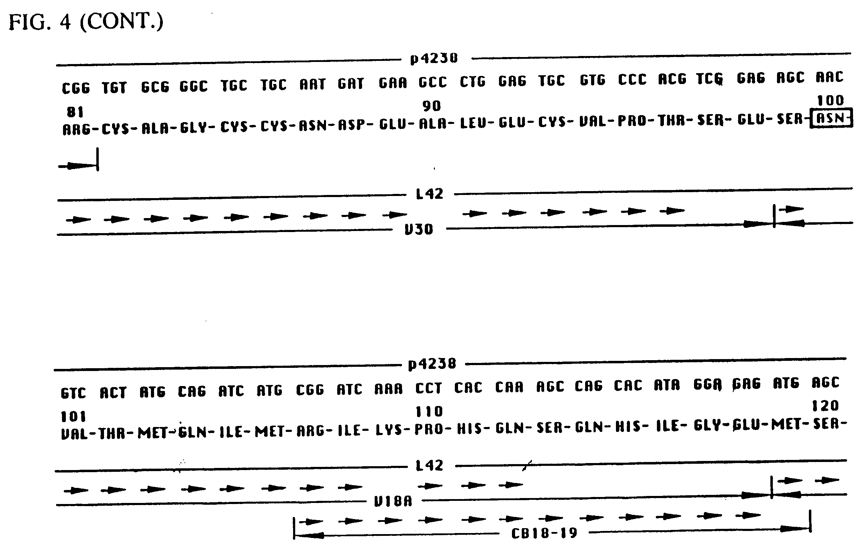

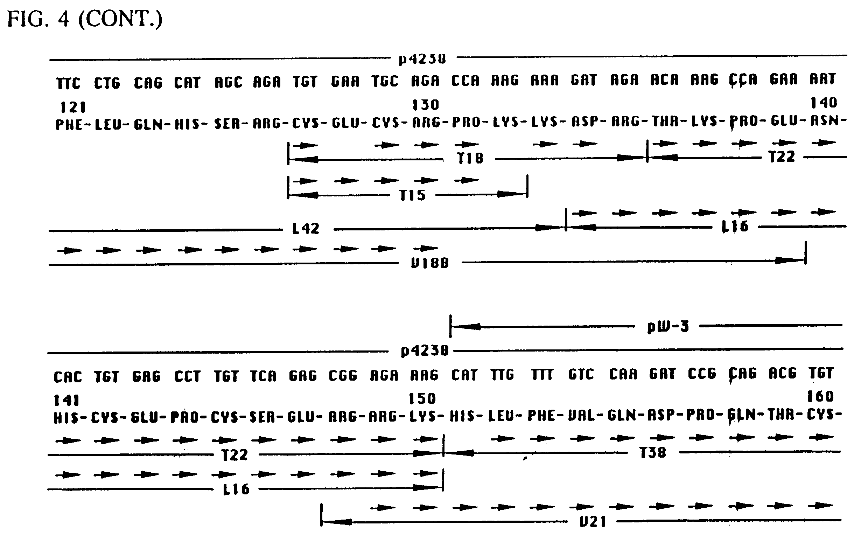

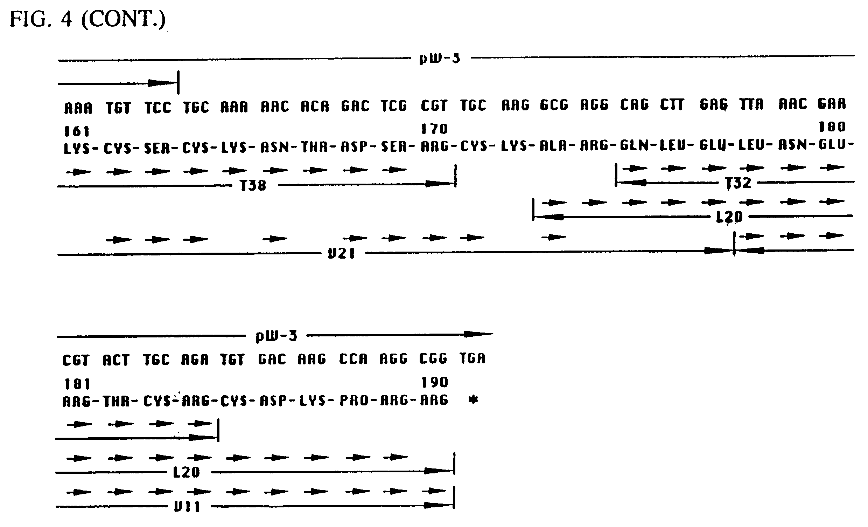

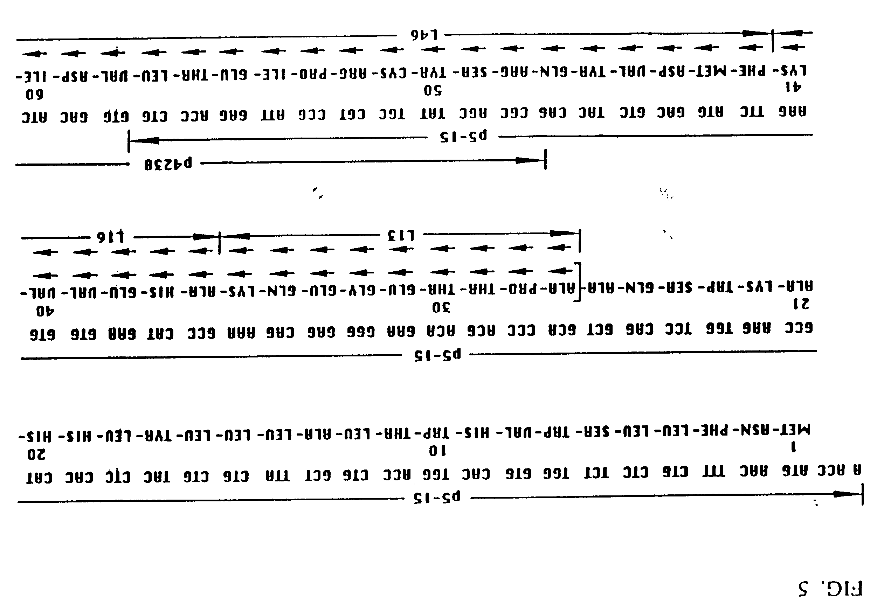

A sample of the A monomer was treated with either the protease trypsin which cleaves polypeptides on the C-terminal side of lysine and arginine residues or Lys C which cleaves polypeptides on the C-terminal side of lysine by procedures well known in the art. The peptides are isolated by reversed phaseHPLC(RP-HPLC). The amino acid sequences of the isolated peptides are determined using the Edman degradation in the ABI gas phase sequenator in conjunction with the ABI 120 A on line phenylthiohydantoin analyzer following manufacturer's instructions. The amino acid sequences are shown in Figure 4.

Reduced and carboxymethylated A monomer is dried and solubilized in about 0.7 M Tris, about pH 7.8, about 6 M guanidinium chloride containing about 0.1% EDTA. V8 protease is added in 0.1 M ammonium bicarbonate buffer, about pH 8.0, and the mixture is incubated for about 48 hr at about 37°C. The protease cleaves predominantly on the carboxyl terminal side of glutamic acid residues. The resulting polypeptides were resolved by C₁₈ RP-HPLC as above.

The reduced and carboxymethylated A subunit protein solution is adjusted to a pH of about 6.8 with 6 N HCl and dithiotreitol is added to a final concentration of 2 M for reduction of any methionine sulfoxide to methionine residues. After about 20 hr of reduction at about 39°C the protein is repurified by C₄ HPLC. The product is dried and cleaved on the carboxyl terminal side of methionine residues by 200 µl of 40 mM cyanogen bromide in about 70 % (v/v) formic acid under an argon atmosphere at about 20°C for about 24 hr in the dark. The cleavage products are resolved by C₁₈ RP-HPLC. The amino acid sequence is shown in Figure 4, see Conn et al., Proc. Natl. Acad. Sci USA 87: 2628-2632 (1990).

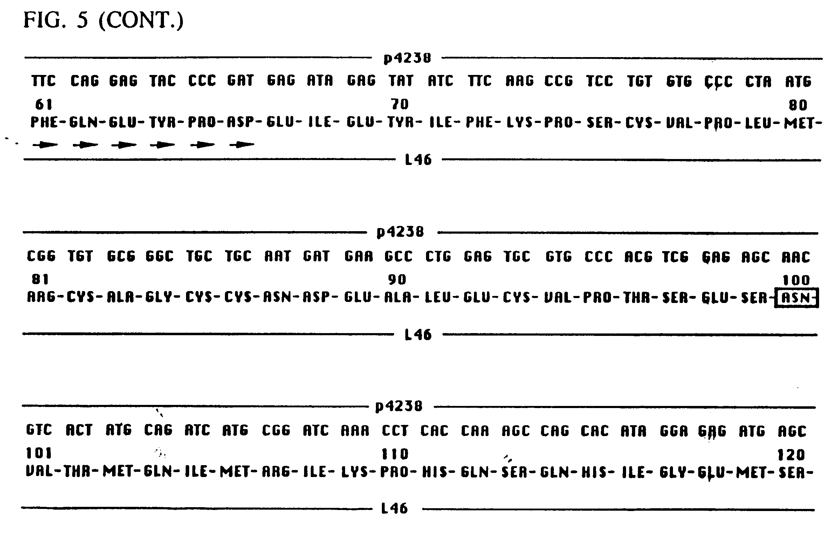

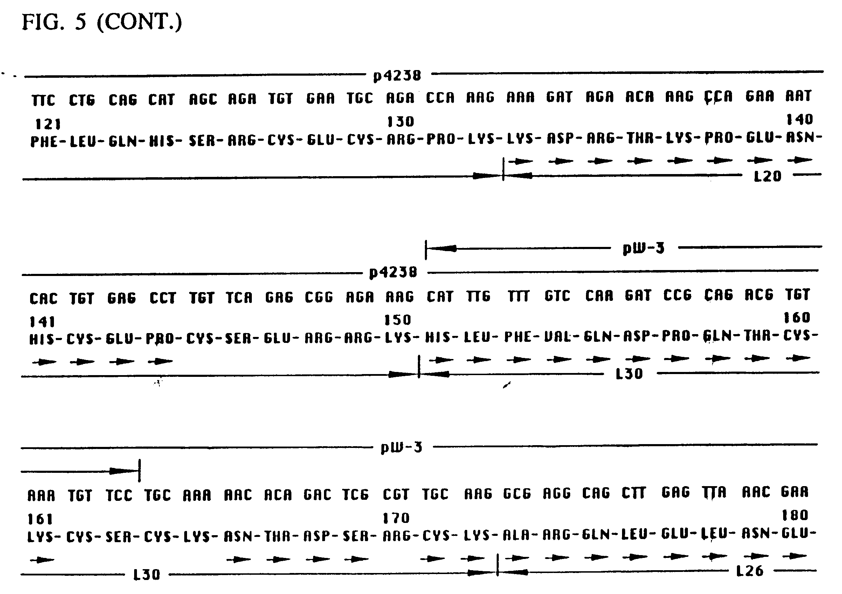

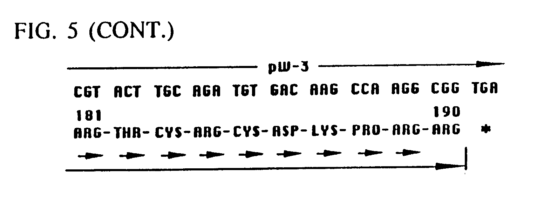

The full length 190 amino acid residue protein translation product of the VEGF II A monomer, which is now known to be identical with the VEGF I A monomer, and its cDNA coding sequence are shown in Fig. 4 and Fig. 5. The mature amino terminus begins at residue 27, immediately following a typical hydrophobic secretory leader sequence. A single potential N-glycosylation site exists at Asn₁₀₀. Most (143 amino acid residues) of the 164 residues of the reduced and carboxymethylated mature subunit including the amino terminus and HPLC reversed phase-purified products of tryptic (T), Lys-C (L), Staphylococcus aureus V8 protease (V8) and cyanogen bromide (CB) cleavages, were determined by direct microsequencing (Applied Biosystems 470A) using a total of 5 µg of protein. All residues identified by amino acid sequencing are denoted by arrows pointing to the right either directly beneath the mature processed sequence following the bracket at residue 27 for the amino terminal determination of the whole subunit or, for residues identified from the polypeptide cleavage products, above the double-headed arrows spanning the lenght of the particular polypeptide. One listed pair of polypeptides, V18A and V18B, was sequenced as a mixture and, therefore, are only confirmatory of the cDNA-deduced amino acid sequence, see Figure 4.

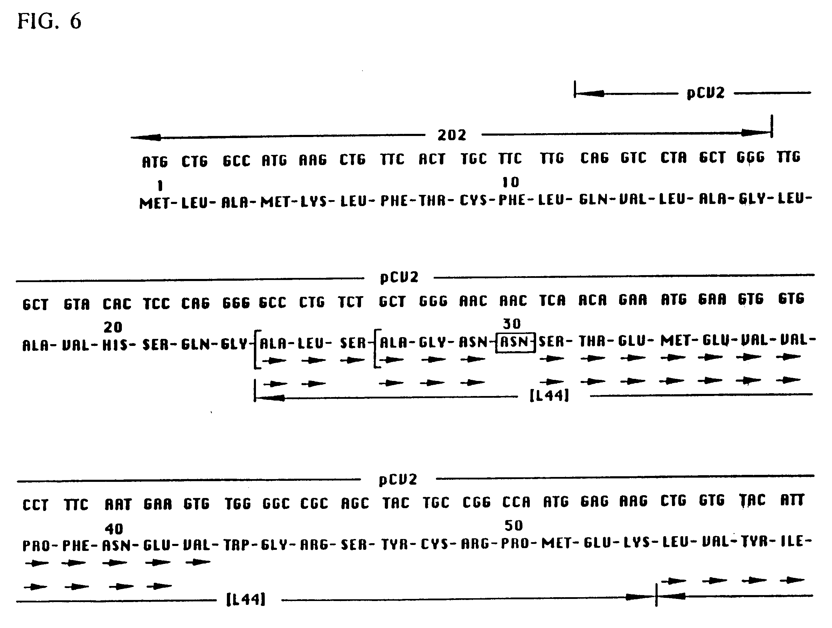

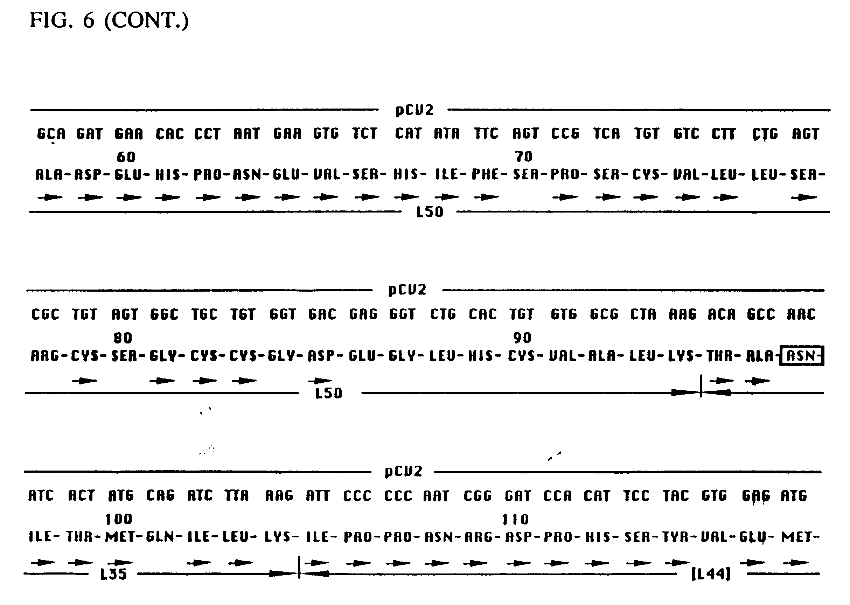

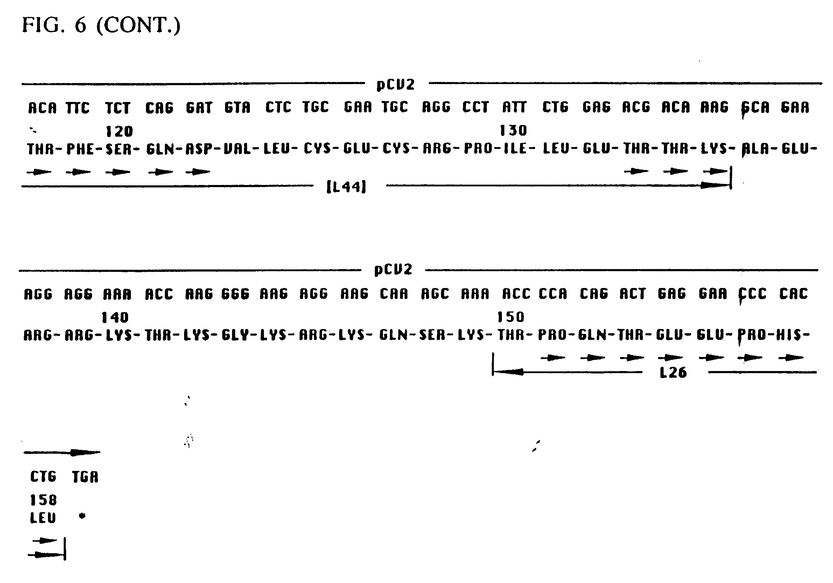

Samples of the reduced and carboxymethylated pure VEGF II A and B monomers were each digested with the Lys-C endoproteinase, which cleaves polypeptides on the C-terminal side of lysine residues. The peptides were isolated by reverse phase HPLC and their amino acid sequences were determined as described above. The locations of the peptides in the final VEGF II A and B sequences are shown in Fig. 5 and Fig. 6, respectively.

The full length coding region of the A monomer is determined from three sets of overlapping cDNA clones. Degenerate oligonucleotide primers based on the amino acid sequences Phe-Met-Asp-Val-Tyr-Gln from polypeptide L42 (residues 42-47) and Cys-Lys-Asn-Thr-Asp from polypeptide T38 (residues 164-168) (see Figs. 4 and 5) were used to PCR amplify the central region of the cDNA for VEGF A chain following the procedure of Saiki et al., Science 230:1350-1354 (1985). A single band migrating at 420 bp was gel purified, digested with SalI, ligated into pGEM3Zf(+) and sequenced. The nucleotide sequence obtained (p4238) was used to design antisense and sense PCR primers to amplify the 5' and 3' ends of the cDNA according to the protocol described by Frohman et al. Proc. Natl. Acad. Sci. USA 85:8998-9002 (1988). These 5' and 3' clones are denoted p5-15 and pW3, respectively. Regions of complete DNA sequences, excluding the primers, determined for each set of clones are indicated by double-headed arrows above the nucleotide sequence. In addition to the cDNA coding the 164 amino acid secreted form identified by protein sequencing, two alternative cDNAs encoding a 120 amino acid and a 188 amino acid form are cloned and sequenced.

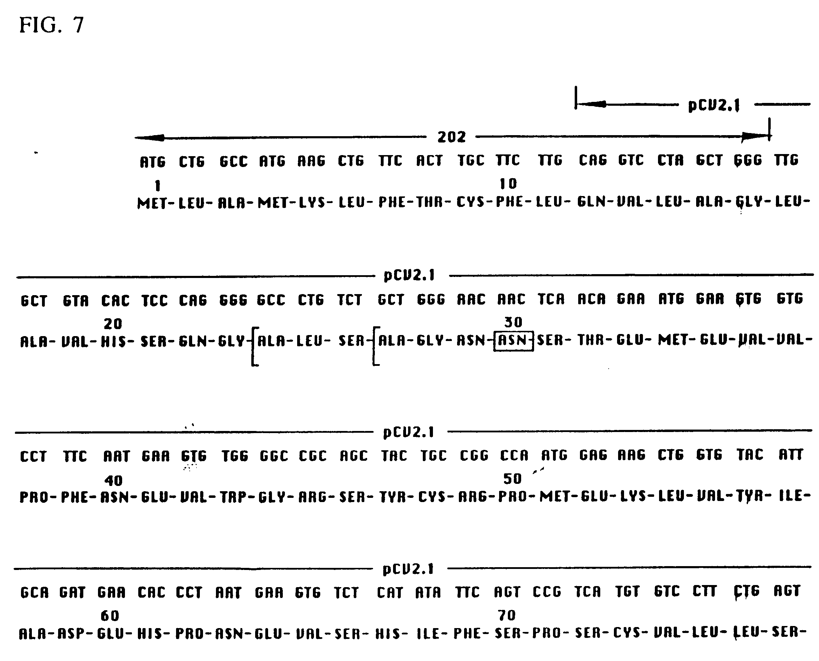

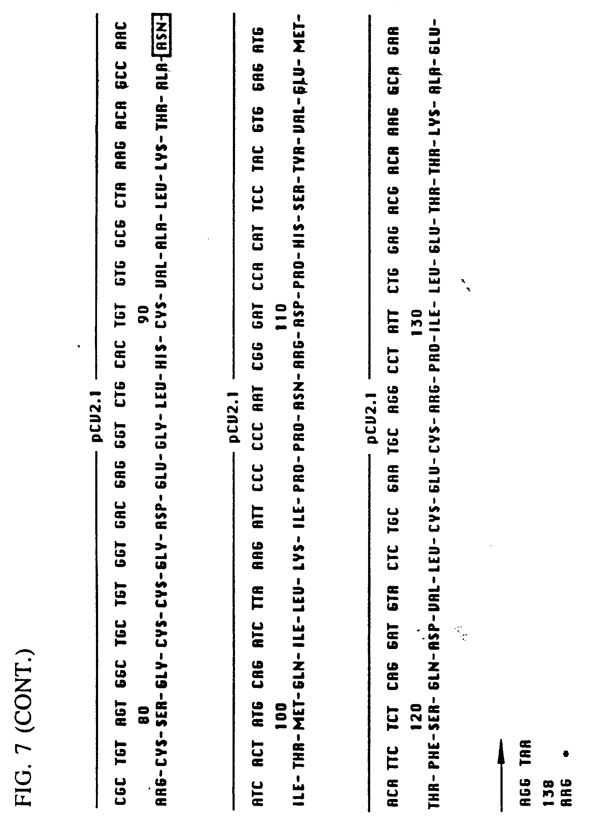

The full length coding region of the B monomer is determined from four sets of overlapping cDNA clones. Degenerate oligonucleotide primers based on the amino acid sequences from polypeptide L50 are used to PCR amplify the central region of the cDNA for VEGF II B monomer following the procedure of Saiki et al., Science 230:1350-1354 (1985). A single band migrating at 108 bp was gel purified, digested with SalI, ligated into pGEM3Zf(+) and sequenced. The nucleotide sequence obtained (pYG) was used to design antisense and sense PCR primers to amplify the 5' and 3' ends of the cDNA according to the protocol described by Frohman et al. Proc. Natl. Acad. Sci. USA 85:8998-9002 (1988). These 5' and 3' clones are denoted p5V2 and p3V2, respectively. Additional 5' end sequences are determined form clone 202 isolated from a cDNA library prepared from GS-9L poly A⁺ RNA. Regions of complete DNA sequences, excluding the primers, determined for each set of clones are indicated by double-headed arrows above the nucleotide sequence. The entire base sequence for the 135 amino acid microheterogeneous B subunit and the 115 amino acid microheterogeneous B subunit are shown in Fig. 6 and Fig. 7.

It is intended that vascular endothelial cell growth factor II exist as a heterodimer consisting of an A subunit and a B subunit.

It is further intended that VEGF homodimers and heterodimers exist as two B subunits. The B subunit may be either the 135 amino acid form or the 115 amino acid form. The A subunit may be either the 188 amino acid form, the 164 amino acid form or the 120 amino acid form. The heterodimers or heterodimer species can be depicted as: A₁₈₈ + B₁₃₅, A₁₈₈ + B₁₁₅, A₁₆₄ + B₁₃₅, A₁₆₄ + B₁₁₅, A₁₂₀ + B₁₃₅, A₁₂₀ + B₁₁₅, and B₁₃₅ + B₁₁₅.

The homodimers can be depicted as: B ₁₃₅ + B₁₃₅, and B₁₁₅ + B₁₁₅. It is also intended that the invention include all of the individual subunit forms of both the B subunits of VEGF II.

It is further intended that the nucleotide sequence for vascular endothelial growth factor II be interpreted to include all codons that code for the appropriate amino acids in the sequence for each of the vascular endothelial growth factor II subunits, as indicated by the degeneracy of the genetic code. It is further intended that the nucleotide sequence and the amino acid sequence for VEGF II subunits include truncated genes or proteins which result in a protein which exhibits biological activity similar to vascular endothelial growth factor II. The scope of the invention is intended to include all naturally occurring mutations and allelic varients and any randomly generated artifical mutants which may change the sequences but do not alter biological activity as determined by the ability to stimulate the division of vascular endothelial cells.

The above described heterodimers, homodimers, subunits and monomers of vascular endothelial growth factor are characterized by being the product of chemical synthetic procedures or of procaryotic or eucaryotic host expression of the DNA sequences as described herein. A monomer is defined as a subunit that cannot form an oligomeric unit. Expression of the recombinant VEGF II genes (recombinant DNA) is accomplished by a number of different host cells which contain at least one of a number of expression vectors. Expression vectors are defined herein as DNA sequences that are required for the transcription of cloned copies of recombinant DNA sequences or genes and the translation of their mRNAs in an appropriate host. Such vectors can be used to express genes in a variety of hosts such as bacteria, blue-green algae, yeast cells, insect cells, plant cells and animal cells, with mammalian cells being preferred. The genes may also be expressed in a number of viruse systems. Specifically designated vectors allow the shuttling of DNA between bacteria-yeast, bacteria-plant or bacteria-animal cells. An appropriately constructed expression vector should contain: an origin of replication for autonomous replication in host cells, selective markers, a limited number of useful restriction enzyme sites, a high copy number, and strong promoters. A promoter is defined as a DNA sequence that directs RNA polymerase to bind to DNA and to initiate RNA synthesis. A strong promoter is one which causes mRNAs to be initiated at high frequency. Expression vectors may include, but are not limited to, cloning vectors, modified cloning vectors, specifically designed plasmids or viruses and cosmids. The expression of mammalian genes in cultured mammalian cells is well known in the art. Sambrook et al., Molecular Cloning, A Laboratory Manual, 2nd Edition, Book 3, Cold Springs Harbor Laboratory Press (1989) and Current Protocols In Molecular Biology, Ausubel et. al. Eds, Greene Publishing Associates and Wiley-Interscience, 1987 and supplements, disclose various mammalian expression vectors and vector systems along with methods for the introduction of recombinant vectors into mammalian cells. The cDNA for the monomeric forms of the A and B subunits can be expressed in a system such as that described by Linemeyer et al., European Patent Application, Publication No. 259,953. The cDNA is incorporated into a commercially available plasmid such as pKK 223-3 (Pharmacia) as modified as by Linemeyer et al. and expressed in E. coli. Other expression systems and host cells are well Known in the art.

The high Cys content and glycoslyation of the A and B subunits along with the structure of the homo-and-heterodimers suggests that expression of biologically active proteins is carried out in animal cells. Expression may be carried out in Chinese hamster ovary (CHO) cells with the cloned VEGF II DNA cotransfected with the gene encoding dihydrofolate reductase (dhfr) into dhfr⁻ CHO cells, see Sambrook et al. Transformants expressing dhfr are selected on media lacking nucleosides and are exposed to increasing concentrations of methotrexate. The dhfr and VEGF II genes are thus coamplified leading to a stable cell line capable of expressing high levels of VEGF II. The plasmid is designed to include either an A subunit and a B subunit or two B subunits. The two cDNAs are operably attached so that the protein produced will be dimeric and will have VEGF II biological activity. Operably attached refers to an appropriate sequential arrangement of nucleotide segments, cDNA segments or genes such that the desired protein will be produced by cells containing an expression vector containing the operably attached genes, cDNA segments or nucleotides.

The expressed proteins (monomers or dimers) are isolated and purified by standard protein purification processes. It is to be understood that the expression vectors capable of expressing heterodimeric forms of VEGF II will contain a DNA sequence which will encode an A subunit and a DNA sequence which will encode a B subunit. Expression vectors capable of expressing homodimeric and heterodimeric forms of VEGF II B may also contain either one or two DNA sequences which encode two B subunits.

The ability of the various species of VEGF II to stimulate the division of vascular endothelial cells makes this protein in all microheterogeneous forms and alternative splicing forms useful as a pharmaceutical agent. The protein as used herein is intended to include all microheterogeneous forms as previously described. The protein can be used to treat wounds of mammals including humans by the administration of the novel protein to patients in need of such treatment.

The novel method for the stimulation of vascular endothelial cells comprises treating a sample of the desired vascular endothelial cells in a nutrient medium with mammalian VEGF II, preferably human or rat, at a concentration of about 1-10 ng/ml. If the vascular endothelial cell growth is conducted in vitro, the process requires the presence of a nutrient medium such as DMEM or a modification thereof and a low concentration of calf or bovine serum such as about 0 to 2% by volume. Preservatives such as antibiotics may also be included; these are well known in the art.

The novel growth factors of this invention are useful for the coverage of artificial blood vessels with vascular endothelial cells. Vascular endothelial cells from the patient would be obtained by removal of a small segment of peripheral blood vessel or capillary-containing tissue and the desired cells would be grown in culture in the presence of VEGF II and any other supplemental components that might be required for growth. After growth of adequate numbers of endothelial cells in culture to cover a synthetic polymeric blood vessel the cells would be plated on the inside surface of the vessel, such as fixed umbilical vein, which is then implanted in the patient. Alternatively, tubular supports are coated in vitro with VEGF II prior to implantation into a patient. Following implantation endothelial cells migrate into and grow on the artificial surface. Prior coating of the artificial vessel either covalently or noncovalently, with proteins such as fibrin, collagen, fibronectin or laminin would be performed to enhance attachment of the cells to the artificial surface. The cell-lined artificial vessel would then be surgically implanted into the patient and, being lined with the patients own cells, would be immunologically compatible. The non-thrombogenic endothelial cell lining should decrease the incidence of clot formation on the surface of the artificial vessel and thereby decrease the tendency of vessel blockage or embolism elsewhere.

The novel proteins are also useful for the production of artificial vessels. Vascular endothelial cells and smooth muscle cells from the patient would be obtained and grown separately in culture. The endothelial cells would be grown in the presence of VEGF II as outlined above. The smooth muscle would be grown in culture by procedures well known in the art. A tubular mesh matrix of a biocompatible polymer (either a synthetic polymer, with or without a coating of proteins, or a non-immunogenic biopolymeric material such as surgical suture thread) would be used to support the culture growth of the smooth muscle cells on the exterior side and vascular endothelial cells on the interior surface. Once the endothelial cells form a confluent monolayer on the inside surface and multiple layers of smooth muscle cells cover the outside, the vessel is implanted into the patient.

The novel peptides can also be used for the induction of tissue repair or growth. The pure VEGF II would be used to induce and promote growth of tissue by inducing vascular growth and /or repair. The peptide can be used either topically for tissue repair or intravascularly for vascular repair. For applications involving neovascularization and healing of surface wounds the formulation would be applied directly at a rate of about 10 ng to about 1 mg/cm²/day. For vascular repair VEGF II is given intraveneously at a rate of about 1 µg to about 100 µg/kg/day of body weight. For internal vascular growth, the formulation would be released directly into the region to be neovascularized either from implanted slow release polymeric material or from slow release pumps or repeated injections. The release rate in either case is about 100 ng to about 100 µg/day/cm³.

For non-topical application the VEGF is administrated in combination with pharamaceutically acceptable carriers or diluents such as, phosphate buffer, saline, phosphate buffered saline, Ringer's solution, and the like, in a pharamaceutical composition, according to standard pharamaceutical practice. For topical application, various pharmaceutical formulations are useful for the administration of the active compound of this invention. Such formulations include, but are not limited to, the following: ointments such as hydrophilic petrolatum or polyethylene glycol ointment; pastes which may contain gums such as xanthan gum; solutions such as alcoholic or aqueous solutions; gels such as aluminum hydroxide or sodium alginate gels; albumins such as human or animal albumins; collagens such as human or animal collagens; celluloses such as alkyl celluloses, hydroxy alkyl celluloses and alkylhydroxyalkyl celluloses, for example methylcellulose, hydroxyethyl cellulose, carboxymethyl cellulose, hydroxypropyl methylcellulose, and hydroxypropyl cellulose; polyoxamers such as Pluronic® Polyols exemplified by Pluronic® F-127; tetronics such as tetronic 1508; and alginates such as sodium alginate.

The following examples illustrate the present invention without, however, limiting the same thereto.

### EXAMPLE 1

### Preparation of Medium Conditioned By GS-9L Cells

GS-9L cells were grown to confluence in 175 cm² tissue culture flasks in Dulbecco's Modified Eagle's Medium/10% newborn calf serum (DMEM/NCS). At confluence the medium was decanted from the flasks, the flasks were washed with calcium and magnesium free phosphate buffered saline (PBS) and the cells were removed by treatment with a 1X solution of trypsin/EDTA. The cells (1 x 10⁸ ) were pelleted by centrifugation, resuspended in 1500 ml of DMEM/5% NCS and plated into a ten level (6000 cm² surface area) cell factory (NUNC). After 72 hours incubation at 37°C in a 5% CO₂ atmosphere the medium was decanted and the cell factories were washed 3 times with PBS. The cells were refed with 1500 ml of a 1:2 mixture of Ham's F-12/DMEM containing 25 mM Hepes, 5 µg/ml insulin, 10 µg/ml transferrin and 1.0 mg/ml bovine serum albumin. This medium was changed with fresh F-12/DMEM after 24 hours and collected every 48 hours after that. The conditioned medium was filtered through a Whatman #1 paper to remove cell debris and stored frozen at -20°C.

### EXAMPLE 2

### Carboxymethyl-Sephadex® Chromatography

GS-9L conditioned medium, from Example 1, was thawed and brought to pH 6.0 with 1 M HCl. Two grams of CM Sephadex C-50 cation exchange (Pharmacia) resin pre-equilibrated in PBS adjusted to pH 6.0 with 1 N HCl is added to 20 liters of conditioned medium. The mixture was stirred at low speed for 24 hours at 4°C. The resin was then allowed to settle and the medium is siphoned off. The remaining resin slurry was packed into a 3.0 cm diameter column and any remaining medium is allowed to drain off. Unbound protein was washed off the column with 0.05 M sodium phosphate, pH 6.0, containing 0.15 M NaCl. Vascular endothelial growth factor activity was eluted from the column with a subsequent wash of 0.05 M sodium phosphate, pH 6.0, containing 0.6 M NaCl.

### EXAMPLE 3

### Concanavalin A (Con A) Lectin Affinity Chromatography

A 0.9 cm diameter column containing about 5 ml of packed Con A agarose (Vector Laboratories) was equilibrated with 0.05 M sodium acetate, pH 6.0, containing 1 mM Ca⁺⁺, 1 mM Mn⁺⁺ and 0.6 M NaCl. The active eluate from the CM Sephadex C-50 column, Example 2, was applied to the Con A agarose and unbound protein was washed from the column with equilibration buffer. The column was then rinsed with three column volumes of 0.05 M sodium acetate, pH 6.0, containing 1 mM Ca⁺⁺, 1 mM Mn⁺⁺ and 0.1 M NaCl. Bound protein was subsequently eluted from the column by application of this buffer supplemented with 0.32 M α-methyl mannoside and 0.28 M α-methyl glucoside.

### EXAMPLE 4

### Polyaspartic Acid WCX HPLC Cation Exchange Chromatography

The active eluate from the Con A column, Example 3, was applied to a 25 cm x 4.6 mm poly(aspartic acid) WCX cation exchange HPLC column (Nest Group) pre-equilibrated in 0.05 M sodium phosphate buffer, pH 6.0. The column was eluted with a linear gradient of 0 to 0.75 M NaCl in this buffer over 60 minutes at a flow rate of 0.75 ml/min collecting 0.75 ml fractions. VEGF II activity present in fractions eluting between approximately 21. 7 and 28.5 ml were pooled as shown by solid horizontal bar in Fig. 1.

### EXAMPLE 5

### Metal Chelate Chromatography

The active fractions eluted from the poly(aspartic acid) WCX column, Example 4, that contain VEGF II were pooled, adjusted to pH 7.0 and loaded onto a 1 x 10 cm column of Pharmacia Chelating Sepharose 6B charged with an excess of copper chloride and equilibrated in 0.05 M sodium phosphate, pH 7.0, containing 2 M NaCl and 0.5 mM imidazole (A buffer). VEGF II was eluted from the column with a gradient from 0-20% B over 10 minutes, 20-35% B over 45 minutes and 35-100% B over 5 minutes at a flow rate of 0.3 ml/min, where B buffer was 0.05 M sodium phosphate, pH 7.0, containing 2 M NaCl and 100 mM imidazole. The active fractions containing VEGF II activity eluting between 12.6 and 22.8 ml of the gradient effluent volume were pooled as shown by the solid horizontal bar in Fig. 2.

### EXAMPLE 6

### Reverse Phase Chromatography

The fractions containing VEGF II activity pooled from the metal chelate column, Example 5 were loaded onto a 4.6 mm x 5 cm Vydac C4 reverse phase HPLC column (5 µm particle size) equilibrated in solvent A (0.1% trifluoroacetic acid (TFA)). The column was eluted with a gradient of 0-30% solvent B over 15 minutes, 30% B for an additional 15 minutes, then 30-45% B over 22.5 minutes and finally 45-100% B over 5.5 minutes where solvent B = A containing 67% acetonitrile. The flow rate was maintained at 0.75 ml/min. The active VEGF II fractions eluting between approximately 32.2 and 37.5 ml of the gradient effluent volume were pooled as shown by the solid horizontal bar in Fig. 3.

### EXAMPLE 7

### Mitogenic Assays

Human umbilical vein endothelial cells (HUVE) were plated on gelatin-coated 48 well tissue culture dishes at a density of 5000 cells/well in 500 µl of Medium 199 containing 20% heat inactivated fetal calf serum (FCS). Samples to be assayed were added at the time of plating. The tissue culture plates are incubated at 37° C for 12 hours and 2 microcuries of tritiated thymidine (NEN, 20 Ci/mmol) was added per ml of assay medium (1.0 µCi/well). The plates were incubated for a further 60 hr, the assay medium was removed and the plates were washed with Hanks balanced salt solution containing 20 mM Hepes, pH 7.5, and 0.5 mg/ml bovine serum albumin. The cells were lysed and the labelled DNA solubilized with 200 µl of a solution containing 2 gm of sodium carbonate and 400 mg sodium hydroxide in 100 ml water. The incorporated radioactivity was determined by liquid scintillation counting.

The concentration of VEGF II which elicited a half-maximal mitogenic response in HUVE cells was approximately 2 ± 1 ng/ml. The glycosaminoglycan heparin, which is required in these assays at a level of 10-100 µg/ml to promote a response to a positive control, acidic fibroblast growth factor, does not enhance mitogenic stimulation of these cells by VEGF II.

### EXAMPLE 8

### Purity And Protein Structure Characterization of VEGF II

Purity of the protein under non-reducing conditions was determined by SDS-PAGE in 12.5% crosslinked gels according to the method of Laemmli, Nature 227: 680-685 (1970). The silver-stained gel contained a single band with an apparent mass of approximately 58 kDa. VEGF II migrated in SDS-PAGE under reducing conditions in 15% crosslinked gels as a broad silver-stained band with apparent molecular mass of approximately 23 kDa.

VEGF II was stored a 4°C in the aqueous trifluoroacetic acid (TFA)/acetonitrile mixture used to elute the homogeneous protein in reversed phase C₄ HPLC chromatography at the final stage of the purification protocol previously described. Aliquots of the purified protein (1-2 µg) were vacuum evaporated to dryness in acid-washed 10 x 75 mm glass tubes and reduced for 2 hours at 50°C in 100 µl of 0.1 M Tris buffer, pH 9.5, and 6 M guanidinium chloride containing 0.1% EDTA and 20 mM dithiothreitol (Calbiochem, Ultrol grade) under an argon atmosphere. The reduced protein was subsequently carboxymethylated for 1 hour at 20°C by the addition of 100 µl of 0.7 M Tris, pH 7.8, containing 0.1 % EDTA, 6 M guanidinium chloride, 9.2 µM unlabeled iodoacetic acid and 50 µCi of iodo[2-¹⁴ C]acetic acid (17.9 mCi/mmole, Amersham). After completion of the carboxymethylation, the mixture was loaded directly onto a 4.6 mm x 5.0 cm Vydac C₄ column which had been preequilibrated in 0.1% TFA. The reduced and carboxymethylated protein was repurified by elution with a 45 minute linear gradient of 0 to 67% (v/v) acetonitrile in 0.1% TFA at a flow rate of 0.75 ml/min and stored in this elution solution at 4°C. The reduced and carboxymethylated protein eluted as two peaks at approximately 25 and 28 ml that were of approximately equal area as determined by monitoring absorbance at 210 nm.

Samples of the two protein subunits isolated after reduction and carboxymethylation were each applied to polybrene-coated glass fiber filters and their N-terminal sequences were determined by Edman degradation in an ABI gas phase microsequencer in conjunction with an ABI 120A on line phenylthiohydantoin analyzer following manufacturers instructions. The peak of absorbance eluting at approximately 28 ml (A subunit) yielded an amino terminal sequence APTTEGEQKAHEVV identical to VEGF 1. The peak of absorbance eluting at approximately 25 ml (B subunit) yielded the N-terminal sequence ALSAGN(X)STEMEVVPFNEV plus a nearly equal amount of a truncated form of the same sequence missing the first three residues. The missing X residue corresponds to an Asn in the cloned sequence. Since this missing Asn occurs in a classical Asn-X-Ser/Thr N-glycosylation sequence it is presumed to be glycosylated. The A and sum of the B chain peptides were recovered in nearly equal amounts supporting the interpretation that the two peptides combine to form an AB heterodimer in VEGF II.

Reduced and carboxymethylated A and B subunits (650 ng each) were each dried by vacuum evaporation in acid-washed 10 x 75 mm glass tubes. Lys C protease (50 ng, Boehringer Mannheim), an enzyme that cleaves on the carboxyl terminal side of lysine residues, was added to each tube in 100 µl of 25 mM Tris, pH 8.5, 0.1 % EDTA. The substrate protein subunits were separately digested at 37°C for 8 hours and the resulting polypeptides resolved by reversed phase HPLC chromatography on a 4.6 mm x 25 cm Vydac C₁₈ column equilibrated in 0.1% TFA. Polypeptides were fractionated by elution with a 2 hour linear gradient of 0-67% acetonitrile in 0.1% TFA at a flow rate of 0.75 ml/min at 20°C. Individual peaks were manually collected and stored in this elution solution at 4°C.

The amino acid sequences of the isolated peptides were then determined using Edman degradation in an ABI gas phase sequenator in conjunction with the ABI 120 A on line phenylthiohydantoin analyzer (Applied Biosystems Int.). The peptide sequences are shown in the following Figs. 5 and 6. The amino acid sequence of Lys C fragment L20 (Fig. 5) demonstrates that the form of VEGF II A subunit in the heterodimer is the 164 amino acid form. The amino acid sequence of Lyc C fragment L26 (Fig. 6) demonstrates that the form of VEGF II B subunit in the heterodimer is the 135 amino acid form.

### EXAMPLE 9

### Cloning and Sequencing of the VEGF II A Monomer

### PCR Amplification. Cloning and Sequencing of P4238

Two degenerate oligonucleotides were synthesized in order to amplify the cDNA encoding the peptide sequences of VEGF A subunit between LysC fragment L 42 and tryptic fragment T38. These oligonucleotides were:
L42.2 5'TTTGTCGACTT[TC]ATGGA[TC]GT[N]TA[TC]CA 3' T383'B
5' CAGAGAATTCGTCGACA[AG]TC[N]GT[AG]TT[TC]TT [AG]CA 3'
where N=ACGT

Poly A⁺ RNA was isolated from GS-9L cells using the Fast Track RNA isolation kit from Invitrogen and the protocol provided. First strand cDNA synthesis was performed as follows;

1 µg of GS-9L RNA was annealled to 1 µg of adapter primer TA17, 5'GACTCGAGTCGACATCGATTTTTTTTTTTTTTTTT 3', by incubating in a volume of 10 µl at 70°C for 5 min. followed by cooling to room temperature. To this reaction was added:
- 3.0 µl: water
- 2.5 µl: 10X buffer (500 mM Tris-HCl pH 8.3, 750 mM KCl, 100mM MgCl₂, 5 mM spermidine)
- 2.5 µl: 100 mM DTT
- 2.5 µl: 10 mM each dATP, dGTP, dCTP, dTTP
- 0.6 µl: 15 units RNasin
- 2.5 µl: 40 mM Na pyrophosphate
- 1.5 µl: 15 units reverse transcriptase
and the reaction was incubated at 42°C for 1 hour, then diluted to 1 ml in 10 mM Tris-HCl l mM EDTA, pH 7.5.

PCR Reactions:

Primary reaction (100 µl)
- 10 µl: 10X buffer from Perkin Elmer Cetus GeneAmp kit
- 16 µl: 1.25 mM each stock of dATP, dCTP, dGTP, and dTTP
- 2 µl: first strand GS9L cDNA
- 2 µl: 50 pMoles L42.2
- 2 µl: 50 pMoles T383'B
- 0.5 µl: 2.5 units Amplitaq DNA polymerase
- 67.5 µl: water
Reaction conditions, 40 cycles of 94°C, 1'; 50°C, 2'30''; 72°C, 2'.

Prep scale secondary reaction:
- 100 µl: 10X buffer
- 160 µl: 1.25 mM each stock of dATP, dCTP, dGTP, and dTTP
- 10 µl: primary PCR reaction
- 20 µl: 500 pMoles L42.2
- 20 µl: 500 pMoles T383'B
- 5 µl: 25 units Amplitaq DNA polymerase
- 685 µl: water
Reaction conditions 94°C, 1'; 55°C, 2'; 72°C, 2'; 30 cycles.

The PCR product was concentrated by Centricon 30 spin columns, purified on a 1% agarose gel, and digested with restriction endonuclease SalI. The SalI fragment was then ligated into SalI cut pGEM3Zf(+). The ligation mix was used to transform E. coli XL-1 blue. Plasmid DNA was isolated from white transformants and sequenced by the dideoxy chain termination method.

### PCR Amplification, Cloning and Sequencing of pW-3

Based on the sequence obtained from the p4238 clones, two specific PCR primers were synthesized; oligo 307 5'TTTGTCGACTCAGAGCGGAGAAAGC 3' and oligo 289 5'TTTGTCGACGAAAATCACTGTGAGC 3'. These primers were used in combination with oligoA 17 5'GACTCGAGTCGACATCG 3' to amplify the cDNA encoding the COOH terminus of VEGF A subunit using the 3' RACE technique described by Frohinan et al., PNAS 85: 8998-9002 (1988).

PCR reactions:

Primary reaction 100 µl
- 10 µl: 10X buffer from Perkin Elmer Cetus GeneAmp kit
- 18 µl: 1.25 mM each stock of dATP, dCTP, dGTP, and dTTP
- 0.35 µl: first strand GS-9L cDNA
- 2 µl: 50 pMoles oligo 289
- 0.5 µl: 2.5 units Amplitaq DNA polymerase
- 67.15µl: water
Reaction conditions 94°C, 1'; 58°C, 2'; 72°C, 2'; 10 cycles then add 50 pMoles A17, then 1 cycle of 94°C, 1'; 58°C, 2'; 72°C, 40' followed by 40 cycles 94°C, 1'; 58°C, 2'; 72°C, 2'.

Prep Scale secondary reaction:
- 60 µl: 10X buffer
- 108 µl: 1.25 mM each stock of dATP, dCTP, dGTP, and dTTP
- 24 µl: primary PCR reaction
- 12 µl: 300 pMoles oligo 307
- 12 µl: 300 pMoles oligo A17
- 3 µl: 15 units Amplitaq DNA polymerase
- 381µl: water
Reaction conditions 94°C, 1'; 58°C, 2'; 72°C, 2'; 30 cycles.

The PCR product was purified on a 1% agarose gel and digested with restriction endonuclease Sa1I. The Sa1I fragment was then ligated into Sa1I cut pGEM3Zf(+). The ligation mix was used to transform E. coli XL-1 blue. Plasmid DNA was isolated from white transformants and sequenced by the dideoxy chain termination method.

### PCR Amplification, Cloning and Sequencing of p5-15

Based on the sequence of p4238 clones, two specific PCR primers were synthesized; oligo 113 5' TTTGTCGACAACACAGGACGGCTTGAAG 3' and oligo 74 5' TTTGTCGACATACTCCTGGAAGATGTCC 3'. These primers were used in combination with oligo A17 5'GACTCGAGTCGACATCG 3' to amplify the cDNA encoding the amino terminus of VEGF A subunit using the 5' RACE technique described by Frohman et al., supra, Oligo 151 was synthesized in order to specifically prime VEGF A subunit cDNA from GS-9L RNA. Oligo 151 is 5'CTTCATCATTGCAGCAGC 3'.

RNA was isolated from GS-9L cells using the Fast Track RNA isolation kit from Invitrogen using the protocol provided. First strand cDNA synthesis was performed as follows;

One µg of GS9L RNA was annealled to 1 µg of oligo 151 by incubating in a volume of 6 µl at 70°C for 5' followed by cooling to room temperature. To this reaction was added:
- 1.5 µl: 10X buffer (500mM Tris-HCl, pH 8.3, 750 mM KCl, 100 mM MgCl₂, 5 mM spermidine)
- 2.5 µl: 10 mM DTT
- 2.5 µl: 10 mM each dATP, dGTP, dCTP, dTTP
- 0.6 µl: 25 units RNasin
- 2.5 µl: 40 mM Na pyrophosphate
- 9.5 µl: 20 units diluted reverse transcriptase
The reaction was incubated at 42°C for 1 hour. Excess oligo 151 was removed by Centricon 100 spin columns and the 5' end of the cDNA was tailed by the addition of dATP and terminal transferase. The tailed cDNA was diluted to a final volume of 150 µl in 10 mM Tris-HCl, 1 mM EDTA, pH 7.5.

PCR Reactions:

Primary reaction (50 µl)
- 5 µl: 10X buffer from Perkin Elmer Cetus GeneAmp Kit
- 8 µl: 1.25 mM each stock of dATP,dCTP,dGTP, and dTTP
- 5 µl: first strand GS-9L cDNA prime with oligo 151 and tailed
- 1 µl: 25 pMoles oligo 113
- 1 µl: 25 pMoles oligo A17
- 1 µl: 10 pMoles oligo TA17
- 0.25 µl: 1.25 units Amplitq DNA polymersase
- 28.75 µl: water
Reaction conditions; 1 cycle 94°C 1'; 50°C 2'; 72°C 40' then 40 cycles of 94°C 1'; 50°C 1'30''; 72°C 2'

Prep scale secondary reaction:
- 60 µl: 10X buffer
- 96 µl: 1.25 mM each stock of dATP, dCTP, dGTP, and dTTP
- 6 µl: primary PCR reaction
- 12 µl: 300 pMoles oligo 74
- 12 µl: 300 pMoles oligo A17
- 3 µl: 15 units Amplitaq DNA polymerase
- 411 µl: water
Reaction conditions 94°C, 1'; 55°C, 2'; 72°C, 2'30 cycles.

The PCR product was concentrated by Centricon 100 spin columns, and digested with restriction endonuclease SalI. The SalI fragment was then ligated into SalI cut pGEM3Zf(+). The ligation mix was used to transform E. coli XL-1 blue. Plasmid DNA was isolated from white transformants and sequenced by the dideoxy chain termination method. The base sequence is shown in Fig. 5.

### Cloning and sequencing of alternative forms of VEGF A cDNA

Based on the sequence obtained from the p5-15 and pW-3 clones, two specific PCR primers were synthesized; oligo 5'C 5' TTTGTCGACAACCATGAACTTTCTGC 3' and oligo 181 5' TTTGTCGACGGTGAGAGGTCTAGTTC 3'. These primers were used together to amplify multiple cDNAs encoding alternative forms of the VEGF A subunit.

Preparative PCR Reaction:
- 50 µl: 10X buffer
- 80 µl: 1.25mM each stock of dATP, dCTP, dGTP, and dTTP
- 10 µl: first strand GS-9L cDNA
- 10 µl: 300pMoles oligo 5'C
- 10 µl: 300pMoles oligo 181
- 2.5 µl: 15 units Amplitaq DNA polymerase
- 337.5 µl: water
Reaction conditions 94°C, 1'; 58°C, 2'; 72°C, 3'; 40 cycles.

The PCR product was extracted with phenol/chloroform, concentrated by Centricon 30 spin columns, precipitated by ethanol, and digested with restriction endonuclease Sal I, and ligated into SalI cut pGEM3Zf(+). The ligation mix was used to transform E.coli XL-1 blue. Plasmid DNA was isolated from white transformants and sequenced by the dideoxy chain termination method. Three sets of clones were identified. Clone #12 encoded the 164 amino acid secreted form of VEGF A subunit identical to that shown in Fig. 4. The 164 amino acid form of VEGF A subunit is that amino acid sequence runing continuously from Ala²⁷ to Arg¹⁹⁰. Clone#14 has a 135 base pair deletion between the second base of the Asn¹⁴⁰ codon and the third base of the Arg¹⁸⁴ codon. This clone thus encodes a 120aa secreted form of the VEGF A subunit with the conversion of Asn¹⁴⁰ to Lys¹⁴⁰. The 120 amino acid form of VEGF A subunit runs from Ala ²⁷ to Asn ¹⁴⁰, which becomes Lys¹⁴⁰ and does not begin until Cys¹⁸⁵, this form also finishes at Arg¹⁹⁰. Clone #16 has a 72 base pair insertion between the second and third base of the Asn¹⁴⁰ codon. This clone thus encodes a 188 amino acid secreted form of the VEGF A subunit with the conversion of Asn¹⁴⁰ to Lys¹⁴⁰. The nucleotide sequence and the deduced amino acid sequence of this insertion is:

Lys Lys Ser Val Arg Gly Lys Gly Lys Gly Gln Lys
  A AAA TCA GTT CGA GGA AAG GGA AAG GGT CAA AAA

Arg Lys Arg Lys Lys Ser Arg Phe Lys Ser Trp Ser
CGA AAG CGC AAG AAA TCC CGG TTT AAA TCC TGG AGC

Val
GT

### EXAMPLE 10

### Cloning and Sequencing of the VEGF II B Subunit

### PCR Amplification, Cloning and Sequencing of pYG

Two degenerate oligonucleotides were synthesized in order to amplify the cDNA encoding the peptide sequences of VEGF II B on Lys C fragment L50. These oligonucleotides were: 
YI 5' TTTGTCGACATA[TC]AT[TCA]GC[N]GA[TC]GA[AG]C 3'
GC 5' TTTGTCGACTC[AG]TC[AG]TT[AG]CA[AG]CA[N]CC 3'
 where N=ACGT

RNA was isolated from GS-9L cells using the Fast Track RNA isolation kit from Invitrogen and the protocol provided. First strand cDNA synthesis was performed as follows;

1 µg of GS-9L poly A⁺RNA was annealled to 1 µg of adapter primer TA17, 5'GACTCGAGTCGACATCGATTTTTTTTTTTTTTTTT 3', by incubating in a volume of 10 µl at 70°C for 5 min. followed by cooling to room temperature. To this reaction was added:
- 3.0 µl: water
- 2.5 µl: 10X buffer (500 mM Tris-HCl, pH 8.3, 750 mM KCl, 100 mM MgCl₂, 5mM spermidine)
- 2.5 µl: 100 mM DTT
- 2.5 µl: 10 mM each dATP, dGTP, dCTP, dTTP
- 0.6 µl: 15 units RNasin
- 2.5 µl: 40 mM Na pyrophosphate
- 1.5 µl: 15 units reverse transcriptase
and the reaction was incubated at 42°C for 1 hour, then diluted to 1 ml in 10 mM Tris-HCl, l mM EDTA, pH 7.5.

PCR Reactions:

Primary reaction (50µl)
- 5 µl: 10X buffer from Perkin Elmer Cetus GeneAmp kit
- 8 µl: 1.25 mM each stock of dATP, dCTP, dGTP, and dTTP
- 1 µl: first strand GS-9L cDNA
- 1 µl: 50 pMoles oligo YI
- 1 µl: 50 pMoles oligo GC
- 0.25 µl: 1.25 units Amplitaq DNA polymerase
- 33.75 µl: water
Reaction conditions, 40 cycles of 94°C, 1'; 50°C, 2'; 72°C, 2'.

Prep scale reaction
- 60 µl: 10X buffer
- 96 µl: 1.25mM each stock of dATP, dCTP, dGTP, and dTTP
- 12 µl: first strand 659L cDNA
- 12 µl: 500pMoles oligo YI
- 12 µl: 500pMoles oligo GC
- 3 µl: 15 units Amplitaq DNA polymerase
- 405 µl: water
Reaction conditions 94°C, 1'; 50°C, 2'; 72°C, 2'40 cycles.

The PCR product was concentrated by Centricon 30 spin columns and digested with restriction endonuclease SalI. The SalI fragment was then ligated into SalI cut pGEM3Zf(+). The ligation mix was used to transform E. coli XL-1 blue. Plasmid DNA was isolated from white transformants and sequenced by the dideoxy chain termination method.

### PCR Amplification, Cloning and Sequencing of p3V2

Based on the sequence obtained from the pYG clones, a specific PCR primer was synthesized; oligo HP 5' TTTGTCGACACACCCTAATGAAGTGTC 3'. This primer was used in combination with oligo A17 5' GACTCGAGTCGACATCG 3' to amplify the cDNA encoding the COOH terminus of the VEGF II B subunit using the 3' RACE technique described by Frohman et al., PNAS 85: 8998-9002 (1988).

Preparative PCR reaction:
- 60 µl: 10X buffer from Perkin Elmer Cetus Gene Amp Kit
- 12 µl: first strand 659L cDNA
- 96 µl: 1.25 mM each of dATP, dCTP, dGTP, dTTP
- 12 µl: 300 pMoles oligo A17
- 12 µl: 300 pMoles oligo HP
- 3 µl: 15units Amplitaq DNA polymerase
- 405 µl: water
Reaction conditions 1 cycle of 94°C, 1'; 58°C, 2'; 72°C, 2'; followed by 40 cycles 94°C, 1', 58°C, 2' and 72°C, 2'.

The PCR product was concentrated by Centricon 30 spin columns, precipitated with ethanol and digested with restriction endonuclease Sa1I. The Sa1I fragment was then ligated into Sa1I cut pGEM3Zf(+). The ligation mix was used to transform E. coli XL-1 blue. Plasmid DNA was isolated from white transformants and sequenced by the dideoxy chain termination method.

### PCR Amplification, Cloning and Sequencing of p5V2

Based on the sequence of pYG clones, two specific PCR primers were synthesized; oligoVL' 5' TTTGTCGACAACAGCGACTCAGAAGG 3' and oligoVS' 5' TTTGTCGACACTGAATATATGAGACAC 3'. These primers were used in combination with oligo A17 5' GACTCGAGTCGACATCG 3' to amplify the cDNA encoding the amino terminus of the VEGF II B subunit using the 5' RACE technique described by Frohman et al., supra. Oligo 151 was synthesized in order to prime cDNA from GS-9L RNA. Oligo 151 is 5' CTTCATCATTGCAGCAGC 3'.

Ploy A⁺RNA was isolated from GS9L cells using the Fast Track RNA isolation kit from Invitrogen using the protocol provided. First strand cDNA synthesis was performed as follows:

One µg of GS9L RNA was annealled to 1 µg of oligo 151 by incubating in a volume of 6 µl at 70°C for 5' followed by cooling to room temperature. To this reaction was added:
- 1.5 µl: 10X buffer (500 mM Tris-HCl, pH 8.3, 750 mM KCl, 100 mM MgCl₂, 5mM spermidine)
- 2.5 µl: 10 mM DTT
- 2.5 µl: 10 mM each dATP, dGTP, dCTP, dTTP
- 0.6 µl: 25 units RNasin
- 2.5 µl: 40 mM Na pyrophosphate
- 9.5 µl: 20 units diluted reverse transcriptase
The reaction was incubated at 42°C for 1 hour.

Excess oligo 151 was removed by Centricon 100 spin columns and the 5' end of the cDNA was tailed by the addition of dATP and terminal transferase. The tailed cDNA was diluted to a final volume of 150 µl in 10 mM Tris-HCl, 1 mM EDTA,pH 7.5

PCR Reactions:

Primary reaction (50 µl)
- 5 µl: 10X buffer from Perkin Elmer Cetus GeneAmp Kit
- 8 µl: 1.25 mM each stock of dATP,dCTP,dGTP, and dTTP
- 5 µl: first strand GS9L cDNA primed with oligo 151 and tailed
- 1 µl: 25 pMoles oligo VL'
- 1 µl: 25 pMoles oligo A17
- 1 µl: 10 pMoles oligo TA17
- 0.25 µl: 1.25 units Amplitq DNA polymersase
- 28.75 µl: water
Reaction conditions; 1 cycle 94°C ,1'; 58°C, 2'; 72°C, 40' then 40 cycles of 94°C, 1'; 58°C, 2'; 72°C, 2'.

Prep scale secondary reaction:
- 100 µl: 10X buffer
- 160 µl: 1.25 mM each stock of dATP, dCTP, dGTP, and dTTP
- 10 µl: primary PCR reaction
- 20 µl: 500 pMoles oligo VS'
- 20 µl: 300 pMoles oligo A17
- 5 µl: 25 units Amplitaq DNA polymerase
- 685 µl: water
Reaction conditions 94°C, 1'; 58°C, 2'; 72°C, 2'30 cycles.

The PCR product was extracted with phenol/chloroform, concentrated by Centricon 30 spin columns, precipitated by ethanol, and digested with restriction endonuclease SalI. The SalI fragment was purified on 4% Nu-Sieve Agarose gel then ligated into SalI cut pGEM3Zf(+). The ligation mix was used to transform E. coli XL-1 blue. Plasmid DNA was isolated from white transformants and sequenced by the dideoxy chain termination method.

### PCR Amplification, Cloning and Sequencing of pCV2 and pCV2.1

Based on the sequences of the p3V2 and p5CV2 clones, two specific PCR primers were synthesized; oligo 5'CV2. 1 5' TTTGTCGAC[N][N]GCAGGTCCTAGCTG 3' and oligo 3'CV2 5' TTTGTCGAC[N][N]CTAATAAATAGAGGG 3'. These primers were used together to amplify the cDNA encoding the VEGF B subunit.

Preparative PCR Reaction:
- 40 µl: 10X buffer
- 64 µl: 1.25 mM each dATP, dTTP, dGTP, dCTP
- 8 µl: first strand GS-9L cDNA
- 8 µl: 200 pMoles 5'CV2.1
- 8 µl: 200 pMoles 3'CV2
- 2 µl: 10units Amplitaq DNA polymerase
- 270 µl: water
Reaction conditions: 94°C, 1', 58°C, 2', 72°C, 2'; 40 cycles.

The PCR product was extracted with phenol/chloroform, concentrated by Centricon 30 spin columns, precipitated by ethanol, and digested with restriction endonuclease Sal I, and ligated into Sal I cut pGEM3Zf(+). The ligation mix was used to transform E. coli XL-1 blue. Plasmid DNA was isolated form white transfromants and sequenced by the dideoxy chain termination method. Two sets of clones were identified, one encoded a 135 amino acid sequence and the other encoded a 115 amino acid sequence, see Figures 6 and 7 respectively.

### cDNA Cloning of VEGF B Subunit

The DNA and protein sequences for the amino terminus of the signal peptide of VEGF B was determined from a cDNA clone isolated from a cDNA library constructed from GS-9L polyA⁺ RNA.

First Strand Synthesis
Anneal 15.6µl (5ug) GS-9L polyA+ RNA and 2.5µl (2.5ug) oligo dT-XbaI primer by heating to 70° C 5' slow cool to room temperature. Add the following:
- 5.5µl: 10X buffer (500 mM Tris-HCl, pH 8.3 (42° C), 750 mM KCl, 100 mM MgCl₂, 5mM spermidine
- 5.5µl: 100mM DTT
- 5.5µl: 10 mM each dATP, dTTP, dCTP, dGTP
- 1.4µl: (55units) RNasin
- 5.5µl: 40mM NaPPi
- 13.5µl: 55units AMV reverse transcriptase
Incubate at 42° C 60'.

Second Strand Synthesis:

Assemble reaction mix
- 50 µl: first strand reaction
- 25 µl: 10X buffer (500 mM Tris-HCl, pH7.2, 850 mM KCL, 30 mM MgCl₂ 1mg/ml BSA, 100 mM (NH₄)₂S0₄
- 7.5 µl: 100 mM DTT
- 25 µl: 1mM NAD
- 6.5 µl: (65units) E. coli DNA PolymeraseI
- 2.5 µl: (2.5units) E. coli DNA Ligase
- 2.5 µl: (2 units) E. coli RNase H
- 135 µl: water
Incubate at 14° C for 2h and then incubate 70° C for 10'. Add 1ul (10 u.nits) T4 DNA Polymerase, incubate at 37° C for 10', add 25 µl 0.2M EDTA an extract with phenol/chloroform, then precipitate by the addition of 0.5 volume of 7.5 M ammonium acetate and 3 volumes of ethanol, collect precipitate and resuspend in 20 µl of 10 mM Tris-HCl, pH 7.5, 1mM EDTA.

cDNA Library Construction

The above cDNA was ligated into EcoR1/ XbaI digested LambdaGEM-4 (Promega Biochemicals) after the addition of EcoR1 linkers and digestion with EcoR1 and XbaI. A cDNA library was amplified from ∼50, 000 independent clones.

Isolation of Rat VEGF B cDNA Clone

The above cDNA library was screen by placque hybridization using pCV2 as a probe. Hybridization conditions were as follows:
5XSSC (1XSSC is 0.15M sodium chloride, 0.015M sodium citrate,
50% Formamide
5X Denhardt's Solution (1% Ficoll, 1% polyvinylpyrrolidone, 1% bovine serum albumin)
0.15 mg/ml salmon sperm DNA
hybridize overnight at 42° C.

Filters were washed 3 times in 2XSSC, 0.1% SDS at room temerature for 5', then 1 time in 1XSSC, 0. 1% SDS at 50C for 30'. Positive clones were identified by autoradiography.

The DNA from phage #202 was digested with restriction endonuclease SpeI and the 1.1kb band ligated into XbaI digested pGEM3Zf(+). The ligation mix was used to transform E.coli XL-1 blue. Plasmid DNA was isolated from white transformants and sequenced by the dideoxy chain termination method. The cDNA sequence and predicted amino acid sequence of the signal peptide are shown in Fig.6 and Fig.7.

The entire nucleotide and amino acid sequence of the 115 amino acid form is shown in Fig. 7. The secreted protein starts at Ala²⁴ and continues to Arg¹³⁸. The entire nucleotide and amino acid sequence of the 135 amino acid form is shown in Fig. 7. The secreted protein starts at Ala²⁴ and continues to Leu¹⁵⁸.

## Claims

1. A mammalian vascular endothelial growth factor II, which comprises:
a) an A subunit, wherein said A subunit is selected from the group consisting of a 188 amino acid mature form of an A subunit, wherein said 188 amino acid mature form has the amino acid sequence shown in Figure 5 from Ala 27 to Arg 190 with the substitution of Asn 140 to Lys 140 and the insertion of the amino acid sequence Lys-Ser-Val-Arg-Gly-Lys-Gly-Lys-Gly-Gln-Lys-Arg-Lys-Arg-Lys-Lys-Ser-Arg-Phe-Lys-Ser-Trp-Ser-Val between Lys 140 and His 141; a 164 amino acid mature form of an A subunit, wherein said 164 amino acid mature form has the amino acid sequence shown in Figure 5 from Ala 27 to Arg 190; and a 120 amino acid mature form of an A subunit, wherein said 120 amino acid mature form has the amino acid sequence shown in Figure 5 from Ala 27 to Arg 190 with the conversion of Asn 140 to Lys 140 and the deletion of the amino acid sequence from His 141 to Arg 184; and
b) a B subunit, wherein said B subunit is a 135 amino acid mature form of a B subunit, wherein said 135 amino acid mature form has the amino acid sequence shown in Figure 6 from Ala 24 to Leu 158, and allelic variants thereof; with said growth factor being substantially free of other proteins and impurities, as well as being mitogenic for mammalian vascular endothelial cells.

2. A mammalian vascular endothelial growth factor II, which comprises:
a) an A subunit, wherein said A subunit is selected from the group consisting of a 188 amino acid mature form of an A subunit, wherein said 188 amino acid mature form has the amino acid sequence shown in Figure 5 from Ala 27 to Arg 190 with the substitution of Asn 140 to Lys 140 and the insertion of the amino acid sequence Lys-Ser-Val-Arg-Gly-Lys-Gly-Lys-Gly-Gln-Lys-Arg-Lys-Arg-Lys-Lys-Ser-Arg-Phe-Lys-Ser-Trp-Ser-Val between Lys 140 and His 141; a 164 amino acid mature form, of an A subunit, wherein said 164 amino acid mature form has the amino acid sequence shown in Figure 5 from Ala 27 to Arg 190; and a 120 amino acid mature form, of an A subunit, wherein said 120 amino acid mature form has the amino acid sequence shown in Figure 5 from Ala 27 to Arg 190 with the conversion of Asn 140 to Lys 140 and the deletion of the amino acid sequence from His 141 to Arg 184; and,
b) a B subunit, wherein said B subunit is a 115 amino acid mature form of a B subunit, wherein said 115 amino acid mature form has the amino acid sequence shown in Figure 7 from amino acid Ala 24 to Arg 138, and allelic variants thereof; with said growth factor being substantially free of other proteins and impurities, as well as being mitogenic for mammalian vascular endothelial cells.

3. A mammalian vascular endothelial growth factor, which comprises either a heterodimer or homodimer of a first and second B subunit, wherein each of said first and second B subunits is independently selected from the group consisting of a 135 amino acid mature form having the amino acid sequence shown in Figure 6 from Ala 24 to Leu 158, a 115 amino acid mature form having the amino acid sequence shown in Figure 7, from Ala 24 to Arg 138, and allelic variants thereof; with said heterodimer or homodimer being substantially free of other proteins and impurities, as well as being mitogenic for mammalian vascular endothelial cells.

4. A process for the isolation of vascular endothelial growth factor as claimed in any one of the preceding claims in substantially pure form which comprises the sequence of steps of:
a. isolation of conditioned growth media;
b. cation exchange chromatography;
c. lectin affinity chromatography;
d. cation exchange high performance liquid chromatography;
e. metal-chelate affinity chromatography;
f. reverse-phase high performance liquid chromatography; and
g. collecting the substantially pure vascular endothelial growth factor.

5. The process of claim 4 which comprises:
a. isolation of conditioned growth media by filtration;
b. cation exchange chromatography with adsorption on carboxymethyl Sephadex® C-50 cation exchange resin at pH 6.0 followed by a rinse with 0.05 M sodium phosphate, pH 6.0, 0. 15 M NaCl and elution with 0.05 sodium phosphate, pH 6.0, containing 0.6 M NaCl;
c. lectin affinity chromatography with adsorption on concanavalin A agarose resin at pH 6.0 followed by elution with 0.05 M sodium acetate, pH 6.0, containing 1 mM CaCl₂, 1 mM MnCl₂, 0.1 M NaCl and 0.32 M alpha-methyl mannoside and 0.28 M alpha-methyl glucoside;
d. cation exchange high performance liquid chromatography with adsorption on Polyaspartic Acid WCX®, equilibrated in 0.05 M sodium phosphate, pH 6.0 followed by elution with about 0 to about 0.75 M NaCl linear gradient;
e. metal-chelate chromatography with adsorption on Pharmacia Chelating Sepharose® 6B charged with excess Cu⁺⁺ followed by elution with a gradient of 0.5 mM to 100 mM imidazole in buffer containing about 0:05 M sodium phosphate, about pH 7.0, about 2 M NaCl;
f. reverse phase high performance liquid chromatography with adsorption on a C₄ column equilibrated in 0.1 % trifluoroacetic acid followed by elution with a gradient of 0 to 67 % (v/v) of acetonitrile in 0.1 % trifluoroacetic; and
g. collecting the substantially pure vascular endothelial growth factor.

6. The process of Claim 4 for the isolation of vascular endothelial growth factor II from GS-9L cell culture fluid.

7. A pharmaceutical composition comprising a pharmaceutical carrier and an effective tissue repairing amount of the purified vascular endothelial growth factor of any one of Claims 1 to 3.

8. The use of the vascular endothelial growth factor of any one of Claims 1 to 3 for the manufacture of a medicament capable of promoting tissue repair.

9. A method for the stimulation of growth of vascular endothelial cells in vitro which comprises treating a sample of the desired endothelial cells in a nutrient medium with vascular endothelial growth factor as claimed in any one of claims 1 to 3 at a concentration of about 0.1-100 ng/ml.

10. A method for preparing synthetic vessels in vitro comprising treating synthetic polymeric vessels with vascular endothelial growth factor as claimed in any one of claims 1 to 3.

11. The use of a synthetic vessel prepared by the method of Claim 10 for the manufacture of a medicament suitable for use as a vessel implant whereby, after implantation, endothelial cells migrate into and grow on the artificial surface.

12. The use of vascular endothelial growth factor as claimed in any one of claims 1 to 3 for the manufacture of a medicament for the stimulation of the growth of vascular endothelial cells in vivo whereby either vascular repair, or neovascularization, or both, is promoted.

13. A purified DNA molecule encoding the 135 amino acid mature form of mammalian vascular endothelial growth factor II, comprising the nucleotide sequence shown in Figure 6.

14. A purified DNA molecule encoding the 115 amino acid mature form of mammalian vascular endothelial growth factor II, comprising the nucleotide sequence shown in Figure 7.

15. Purified DNA molecules encoding a mammalian vascular endothelial growth factor II, which comprises:
a) an A subunit DNA molecule, wherein said A subunit is selected from the group of DNA molecules consisting of: a DNA molecule encoding a 188 amino acid mature form, wherein said DNA molecule encodes a 188 amino acid form, a 164 amino acid form and a 120 amino acid form, whereby said 188 amino acid mature form has the amino acid sequence shown in Figure 5 from Ala 27 to Arg 190 with the substitution of Asn 140 to Lys 140 and the insertion of the amino acid sequence Lys-Ser-Val-Arg-Gly-Lys-Gly-Lys-Gly-Gln-Lys-Arg-Lys-Arg-Lys-Lys-Ser-Arg-Phe-Lys-Ser-Trp-Ser-Val between Lys 140 and His 141; a 164 amino acid mature form has the amino acid sequence shown in Figure 5 from Ala 27 to Arg 190; and a 120 amino acid mature form has the amino acid sequence shown in Figure 5 from Ala 27 to Arg 190 with the conversion of Asn 140 to Lys 140 and the deletion of the amino acid sequence from His 141 to Arg 184; and
b) a B subunit DNA molecule, wherein said B subunit is a selected from the group of DNA molecules consisting of: a DNA molecule encoding molecule encoding the 135 amino acid mature form shown in Figure 6 and a DNA molecule encoding the 115 amino acid mature form shown in Figure 7.

16. A purified DNA molecule encoding heterodimeric vascular endothelial growth factor II which consists of a DNA molecule comprising an A subunit and a DNA molecule comprising a B subunit, selected from the DNA molecules of claim 15.

17. A vector containing a DNA molecule as claimed in any one of claims 13, 14 and 16.

18. A host cell transformed by a DNA vector of claim 17.

19. A process for the preparation of vascular endothelial growth factor II comprising culturing the transformed host cell of claim 18 under conditions suitable for the expression of vascular endothelial growth factor II and recovering vascular endothelial growth factor II.

20. Vascular endothelial growth factor II made by the process of claim 19.

## Patentansprüche

1. Säuger-Gefäßendothelzell-Wachstumsfaktor II, umfassend:
a) eine A-Untereinheit, wobei die A-Untereinheit aus der Gruppe, bestehend aus einer reifen Form einer A-Untereinheit mit 188 Aminosäuren, wobei die reife Form mit 188 Aminosäuren die in Figur 5 dargestellte Aminosäuresequenz von Ala 27 bis Arg 190 mit der Substitution von Asn 140 durch Lys 140 und der Insertion der Aminosäuresequenz Lys-Ser-Val-Arg-Gly-Lys-Gly-Lys-Gly-Gln-Lys-Arg-Lys-Arg-Lys-Lys-Ser-Arg-Phe-Lys-Ser-Trp-Ser-Val zwischen Lys 140 und His 141 aufweist, einer reifen Form einer A-Untereinheit mit 164 Aminosäuren, wobei die reife Form mit 164 Aminosäuren die in Figur 5 dargestellte Aminosäuresequenz von Ala 27 bis Arg 190 aufweist, und einer reifen Form einer A-Untereinheit mit 120 Aminosäuren, wobei die reife Form mit 120 Aminosäuren die in Figur 5 dargestellte Aminosäuresequenz von Ala 27 bis Arg 190 mit der Überführung von Asn 140 in Lys 140 und der Deletion der Aminosäuresequenz von His 141 bis Arg 184 aufweist, ausgewählt ist, und
b) eine B-Untereinheit, wobei die B-Untereinheit eine reife Form einer B-Untereinheit mit 135 Aminosäuren ist, wobei die reife Form mit 135 Aminosäuren die in Figur 6 dargestellte Aminosäuresequenz von Ala 24 bis Leu 158 aufweist,
und allele Varianten davon; wobei der Wachstumsfaktor im wesentlichen frei von anderen Proteinen und Verunreinigungen sowie mitogen für Säuger-Gefäßendothelzellen ist.

2. Säuger-Gefäßendothelzell-Wachstumsfaktor II, umfassend:
a) eine A-Untereinheit, wobei die A-Untereinheit aus der Gruppe, bestehend aus einer reifen Form einer A-Untereinheit mit 188 Aminosäuren, wobei die reife Form mit 188 Aminosäuren die in Figur 5 dargestellte Aminosäuresequenz von Ala 27 bis Arg 190 mit der Substitution von Asn 140 durch Lys 140 und der Insertion der Aminosäuresequenz Lys-Ser-Val-Arg-Gly-Lys-Gly-Lys-Gly-Gln-Lys-Arg-Lys-Arg-Lys-Lys-Ser-Arg-Phe-Lys-Ser-Trp-Ser-Val zwischen Lys 140 und His 141 aufweist, einer reifen Form einer A-Untereinheit mit 164 Aminosäuren, wobei die reife Form mit 164 Aminosäuren die in Figur 5 dargestellte Aminosäuresequenz von Ala 27 bis Arg 190 aufweist, und einer reifen Form einer A-Untereinheit mit 120 Aminosäuren, wobei die reife Form mit 120 Aminosäuren die in Figur 5 dargestellte Aminosäuresequenz von Ala 27 bis Arg 190 mit der Überführung von Asn 140 in Lys 140 und der Deletion der Aminosäuresequenz von His 141 bis Arg 184 aufweist, ausgewählt ist, und
b) eine B-Untereinheit, wobei die B-Untereinheit eine reife Form einer B-Untereinheit mit 115 Aminosäuren ist, wobei die reife Form mit 115 Aminosäuren die in Figur 7 dargestellte Aminosäuresequenz von Ala 24 bis Arg 138 aufweist,
und allele Varianten davon; wobei der Wachstumsfaktor im wesentlichen frei von anderen Proteinen und Verunreinigungen sowie mitogen für Säuger-Gefäßendothelzellen ist.

3. Säuger-Gafäßendothelzell-Wachstumsfakor, umfassend entweder ein Heterodimer oder Homodimer einer ersten und zweiten B-Untereinheit, wobei jede der ersten und zweiten B-Untereinheiten unabhängig aus der Gruppe, bestehend aus einer reifen Form mit 135 Aminosäuren, welche die in Figur 6 dargestellte Aminosäuresequenz von Ala 24 bis Leu 158 aufweist, einer reifen Form mit 115 Aminosäuren, welche die in Figur 7 dargestellte Aminosäuresequenz von Ala 24 bis Arg 138 aufweist, und allelen Varianten davon, ausgewählt ist; wobei das Heterodimer oder Homodimer im wesentlichen frei von anderen Proteinen und Verunreinigungen sowie mitogen für Säuger-Gefäßendothelzellen ist.

4. Verfahren zur Isolierung von Gefäßendothelzell-Wachstumsfaktor nach irgendeinem der vorhergehenden Ansprüche in im wesentlichen reiner Form, umfassend die Folge der Schritte:
a. Isolierung von konditionierten Wachstumsmedien;
b. Kationenaustauschchromatographie;
c. Lektin-Affinitätschromatographie;
d. Kationenaustausch-Hochleistungsflüssigkeitschromatographie;
e. Metallchelat-Affinitätschromatographie;
f. Umkehrphasen-Hochleistungsflüssigkeitschromatographe; und
g. Gewinnung des im wesentlichen reinen Gefäßendothelzell-Wachstumsfaktors.

5. Verfahren nach Anspruch 4, umfassend:
a. Isolierung von konditionierten Wachstumsmedien durch Filtration;
b. Kationenaustauschchromatographie mit Adsorption an Carboxymethyl-Sephadex® C-50-Kationenaustauschharz bei pH 6,0, gefolgt von einer Spülung mit 0,05 M Natriumphosphat, pH 6,0, 0,15 M NaCl und Elution mit 0,05 M Natriumphosphat, pH 6,0, enthaltend 0,6 M NaCl;
c. Lektin-Affinitätschromatographie mit Adsorption an Concanavalin A-Agaroseharz bei pH 6,0, gefolgt von Elution mit 0,05 M Natriumacetat, pH 6,0, enthaltend 1 mM CaCl₂, 1mM MnCl₂, 0,1 M NaCl und 0,32 M α-Methylmannosid und 0,28 M α-Methylglucosid;
d. Kationenaustausch-Hochleistungsflüssigkeitschromatographie mit Adsorption an Polyasparaginsäure WCX®, äquilibriert in 0,05 M Natriumphosphat, pH 6,0, gefolgt von Elution mit einem linearen Gradienten mit etwa 0 bis etwa 0,75 M NaCl;
e. Metallchelat-Chromatographie mit Adsorption an Pharmacia Chelating Sepharose® 6B, beladen mit überschüssigem Cu⁺⁺, gefolgt von Elution mit einem Gradienten von 0,5 mM bis 100 mM Imidazol in Puffer, der etwa 0,05 M Natriumphosphat, etwa pH 7,0, etwa 2 M NaCl enthält;
f. Umkehrphasen-Hochleistungsflüssigkeitschromatographie mit Adsorption an eine in 0,1%iger Trifluoressigsäure äquilibrierte C₄-Säule, gefolgt von Elution mit einem Gradienten von 0 bis 67% (Vol./Vol.) Acetonitril in 0,1%iger Trifluoressigsäure; und
g. Gewinnung des im wesentlichen reinen Gefäßendothelzell-Wachstumsfaktors.

6. Verfahren nach Anspruch 4 zur Isolierung von Gefäßendothelzell-Wachstumsfaktor II aus GS-9L-Zellkulturflüssigkeit.

7. Pharmazeutische Zusammensetzung, umfassend einen pharmazeutischen Träger und eine wirksame, gewebe-wiederherstellende Menge des gereinigten Gefäßendothelzell-Wachstumsfaktors nach irgendeinem der Ansprüche 1 bis 3.

8. Verwendung des Gefäßendothelzell-Wachstumsfaktors nach irgendeinem der Ansprüche 1 bis 3 zur Herstellung eines Medikaments, welches zur Förderung der Wiederherstellung von Gewebe in der Lage ist.

9. Verfahren zur Stimulierung des Wachstums von Gefäßendothelzellen in vitro, welches umfaßt, daß eine Probe der gewünschten Endothelzellen in einem Nährmedium mit Gefäßendothelzell-Wachstumsfaktor nach irgendeinem der Ansprüche 1 bis 3 in einer Konzentration von etwa 0,1-100 ng/ml behandelt wird.

10. Verfahren zur Herstellung von synthetischen Gefäßen in vitro, welches umfaßt, daß synthetische polymere Gefäße mit Gefäßendothelzell-Wachstumsfaktor nach irgendeinem der Ansprüche 1 bis 3 behandelt werden.

11. Verwendung eines synthetischen Gefäßes, welches nach dem Verfahren von Anspruch 10 hergestellt wurde, zur Herstellung eines Medikaments, welches sich zur Verwendung als Gefäßimplantat eignet, wodurch nach Implantation Endothelzellen in die künstliche Oberfläche einwandern und darauf wachsen.

12. Verwendung des Gefäßendothelzell-Wachstumsfaktors nach irgendeinem der Ansprüche 1 bis 3 zur Herstellung eines Medikaments zur Stimulierung des Wachstums von Gefäßendothelzellen in vivo, durch welches entweder Gefäßwiederherstellung oder Gefäßneubildung oder beides gefördert wird.

13. Gereinigtes DNA-Molekül, kodierend für die reife Form von Säuger-Gefäßendothelzell-Wachstumsfaktor II mit 135 Aminosäuren, welches die in Figur 6 dargestellte Nukleotidsequenz umfaßt.

14. Gereinigtes DNA-Molekül, kodierend für die reife Form von Säuger-Gefäßendothelzell-Wachstumsfaktor II mit 115 Aminosäuren, welches die in Figur 7 dargestellte Nukleotidsequenz umfaßt.

15. Gereinigte DNA-Moleküle, die für einen Säuger-Gefäßendothelzell-Wachstumsfaktor II kodieren, umfassend:
a) ein A-Untereinheits-DNA-Molekül, wobei die A-Untereinheit aus der Gruppe von DNA-Molekülen, bestehend aus einem für eine reife Form mit 188 Aminosäuren kodierenden DNA-Molekül, wobei das DNA-Molekül für eine Form mit 188 Aminosäuren, eine Form mit 164 Aminosäuren und eine Form mit 120 Aminosäuren kodiert, wobei die reife Form mit 188 Aminosäuren die in Figur 5 dargestellte Aminosäuresequenz von Ala 27 bis Arg 190 mit der Substitution von Asn 140 durch Lys 140 und der Insertion der Aminosäuresequenz Lys-Ser-Val-Arg-Gly-Lys-Gly-Lys-Gly-Gln-Lys-Arg-Lys-Arg-Lys-Lys-Ser-Arg-Phe-Lys-Ser-Trp-Ser-Val zwischen Lys 140 und His 141 aufweist, eine reife Form mit 164 Aminosäuren die in Figur 5 dargestellte Aminosäuresequenz von Ala 27 bis Arg 190 aufweist, und eine reife Form mit 120 Aminosäuren die in Figur 5 dargestellte Aminosäuresequenz von Ala 27 bis Arg 190 mit der Überführung von Asn 140 in Lys 140 und der Deletion der Aminosäuresequenz von His 141 bis Arg 184 aufweist, ausgewählt ist, und
b) ein B-Untereinheits-DNA-Molekül, wobei die B-Untereinheit aus der Gruppe von DNA-Molekülen ausgewählt ist, die aus einem DNA-Molekül, kodierend für ein Molekül, das für die in Figur 6 dargestellte reife Form mit 135 Aminosäuren kodiert, und einem DNA-Molekül, das für die in Figur 7 dargestellte reife Form mit 115 Aminosäuren kodiert, besteht.

16. Gereinigtes DNA-Molekül, kodierend für heterodimeren Gefäßendothelzell-Wachstumsfaktor II, das aus einem DNA-Molekül, welches eine A-Untereinheit umfaßt, und einem DNA-Molekül, welches eine B-Untereinheit umfaßt, ausgewählt aus den DNA-Molekülen von Anspruch 15, besteht.

17. Vektor, enthaltend ein DNA-Molekül nach irgendeinem der Ansprüche 13, 14 und 16.

18. Wirtszelle, transformiert mit einem DNA-Vektor nach Anspruch 17.

19. Verfahren zur Herstellung von Gefäßendothelzell-Wachstumsfaktor II, umfassend die Kultivierung der transformierten Wirtszelle nach Anspruch 18 unter Bedingungen, die zur Expression von Gefäßendothelzell-Wachstumsfaktor II geeignet sind, und die Gewinnung von Gefäßendothelzell-Wachstumsfaktor II.

20. Gefäßendothelzell-Wachstumsfaktor II, hergestellt nach dem Verfahren von Anspruch 19.

## Revendications

1. Un facteur II de croissance de cellules vasculaires endothéliales de mammifères, qui comprend :
a) une sous-unité A, ladite sous-unité A étant choisie dans le groupe constitué d'une forme mature de 188 acides aminés d'une sous-unité A dans laquelle ladite forme mature de 188 acides aminés présente la séquence d'acides aminés représentée sur la figure 5 à partir de Ala 27 jusqu'à Arg 190, avec la substitution de Asn 140 en Lys 140 et l'insertion de la séquence d'acides aminés Lys-Ser-Val-Arg-Gly-Lys-Gly-Lys-Gly-Gln-Lys-Arg-Lys-Arg-Lys-Lys-Ser-Arg-Phe-Lys-Ser-Trp-Ser-Val entre Lys 140 et His 141 ; une forme mature de 164 acides aminés d'une sous-unité A, dans laquelle ladite forme mature de 164 acides aminés possède la séquence d'acides aminés représentée sur la figure 5 de Ala 27 à Arg 190 ; et une forme mature de 120 acides aminés d'une sous-unité A, dans laquelle ladite forme mature de 120 acides aminés possède la séquence d'acides aminés représentée sur la figure 5 à partir de Ala 27 à Arg 190, avec la conversion de Asn 140 en Lys 140 et la délétion de la séquence d'acides aminés de His 141 à Arg 184 ; et
b) une sous-unité B, ladite sous-unité B étant une forme mature de 135 acides aminés d'une sous-unité B, dans laquelle ladite forme mature de 135 acides aminés possède la séquence acide aminé représentée sur la figure 6, à partir de Ala 24 à Leu 158, et des variantes alléliques de celle-ci, ledit facteur de croissance étant essentiellement dépourvu d'autres protéines et d'autres impuretés, ainsi qu'étant mitogénique pour les cellules vasculaires endothéliales de mammifères.

2. Un facteur II de la croissance de cellules vasculaires endothéliales de mammifères, qui comprend:
a) une sous-unité A, ladite sous-unité A étant choisie dans le groupe constitué d'une forme mature de 188 acides aminés d'une sous-unité A dans laquelle ladite forme mature de 188 acides aminés présente la séquence d'acides aminés représentée sur la figure 5 à partir de Ala 27 jusqu'à Arg 190, avec la substitution de Asn 140 en Lys 140 et l'insertion de la séquence d'acides aminés Lys-Ser-Val-Arg-Gly-Lys-Gly-Lys-Gly-Gln-Lys-Arg-Lys-Arg-Lys-Lys-Ser-Arg-Phe-Lys-Ser-Trp-Ser-Val entre Lys 140 et His 141 ; une forme mature de 164 acides aminés d'une sous-unité A, dans laquelle ladite forme mature de 164 acides aminés possède la séquence d'acides aminés représentée sur la figure 5 de Ala 27 à Arg 190 ; et une forme mature de 120 acides aminés d'une sous-unité A, dans laquelle ladite forme mature de 120 acides aminés possède la séquence d'acides aminés représentée sur la figure 5 de Ala 27 à Arg 190 ; avec la conversion de Asn 140 en Lys 140 et la délétion de la séquence d'acides aminés de His 141 à Arg 184 ; et
b) une sous-unité B, ladite sous-unité B étant une forme mature de 115 acides aminés d'une sous-unité B, dans laquelle ladite forme mature de 115 acides aminés possède la séquence acide aminé représentée sur la figure 7, à partir de Ala 24 à Arg 138, et des variantes alléliques de celle-ci ; ledit facteur de croissance étant essentiellement dépourvu d'autres protéines et d'autres impuretés, ainsi qu'étant mitogénique pour les cellules vasculaires endothéliales de mammifères.

3. Un facteur de croissance vasculaire endothéliale de mammifères, qui comprend un hétérodimère ou bien un homodimère d'une première et d'une seconde sous-unités B, où chacune desdites première et seconde sous-unités B est choisie indépendamment dans le groupe constitué d'une forme mature de 135 acides aminés, ayant la séquence d'acides aminés représentée sur la figure 6 de Ala 24 jusqu'à Leu 158, une forme mature de 115 acides aminés ayant la séquence d'acides aminés représentés sur la figure 7, de Ala 24 à Arg 138 et des variantes alléliques de celle-ci, ledit hétérodimère ou ledit homodimère étant essentiellement dépourvu d'autres protéines et impuretés, ainsi qu'étant mitogéniques pour des cellules vasculaires endothéliales de mammifères.

4. Un procédé pour l'isolement du facteur de croissance vasculaire endothéliale tel que revendiqué dans l'une quelconque des revendications précédentes, sous forme essentiellement pure, qui comprend la séquence des étapes de :
a. isolement de milieux de croissance conditionnés,
b. chromatographie par échange de cations,
c. chromatographie par affinité pour la lectine,
d. chromatographie liquide de haute performance par échange de cations,
e. chromatographie par affinité pour chélate de métal,
f. chromatographie liquide de haute performance en phase inverse, et
g. recueil du facteur de croissance vasculaire endothéliale, essentiellement pur.

5. Le procédé de la revendication 4, qui comporte :
a. l'isolement des milieux de croissance conditionnés par filtration ;
b. chromatographie par échange de cations avec adsorption sur une résine échangeuse de cations de type carboxyméthyl Sephadex ® C-50 à pH 6,0, suivi par un rinçage avec du phosphate de sodium 0,05 M, pH 6,0, du NaCl 0,15 M et élution avec du phosphate de sodium 0,05 M, pH 6,0, renfermant du NaCl 0,6 M,
c. chromatographie par affinité pour la lectine avec adsorption sur résine d'agarose de type concanavalin A à pH 6,0 suivi par élution avec de l'acétate de sodium 0,05 M, pH 6,0, renfermant du CaCl₂ 1 mM, du MnCl₂ 1 mM, du NaCl 0,1 M et de l'α-méthylmannoside 0,32 M et de l'α-méthylglucoside 0,28 M;
d. chromatographie liquide de haute performance par échange de cations avec adsorption sur l'acide polyaspartique WCX ®, équilibré dans du phosphate de sodium 0,05 M, pH 6,0 suivi par élution avec environ 0 à environ 0,75 M de gradient linéaire NaCl;
e. chromatographie de chélate de métal avec adsorption sur Pharmacia Chelating Sepharose® 6B chargé avec du Cu++ en excès suivi par une élution avec un gradient de 0,5 mM à 100 mM d'imidazole dans un tampon renfermant environ 0,05 M de phosphate de sodium, environ pH 7,0, environ 2 M de NaCl ;
f. chromatographie liquide de haute performance en phase inverse, avec adsorption sur une colonne C₄ équilibrée dans 0,1 % d'acide trifluoroacétique, suivi par élution avec un gradient de 0 à 67 % (v/v) d'acétonitrile dans 0,1 % d'acide trifluoroacétique ; et
g. recueil du facteur de croissance vasculaire endothéliale, essentiellement pur.

6. Le procédé de la revendication 4 pour l'isolement du facteur II de croissance vasculaire endothéliale d'un fluide de culture de cellules GS-9L.

7. Une composition pharmaceutique comportant un support pharmaceutique et une quantité efficace pour la réparation de tissus de facteur de croissance vasculaire endothéliale, purifié, de l'une quelconque des revendications 1 à 3.

8. L'utilisation du facteur de croissance vasculaire endothéliale de l'une quelconque des revendications 1 à 3 pour la fabrication d'un médicament apte à promouvoir la réparation des tissus.

9. Un procédé pour la stimulation de la croissance de cellules vasculaires endothéliales in vitro, qui comprend le traitement d'un échantillon des cellules endothéliales souhaitées dans un milieu nutritif avec un facteur de croissance vasculaire endothéliale tel que revendiqué dans l'une quelconque des revendications 1 à 3, selon une concentration d'environ 0,1-100 ng/ml.

10. Un procédé pour la préparation de vaisseaux synthétiques in vitro, comprenant le traitement des vaisseaux polymères synthétiques avec le facteur de croissance vasculaire endothéliale tel que revendiqué dans l'une quelconque des revendications 1 à 3.

11. Utilisation d'un vaisseau synthétique préparé par la technique de la revendication 10 pour la fabrication d'un médicament approprié pour être utilisé en tant qu'implant pour vaisseau, de façon que, après implantation, les cellules endothéliales migrent dans et croissent sur la surface artificielle.

12. Utilisation du facteur de croissance vasculaire endothéliale tel que revendiqué dans l'une quelconque des revendications 1 à 3, pour la fabrication d'un médicament pour la stimulation de la croissance des cellules vasculaire endothéliales in vivo, de façon à promouvoir une réparation vasculaire ou une néovascularisation ou bien les deux.

13. Une molécule purifiée d'ADN encodant la forme mature de 135 acides aminés du facteur II de croissance vasculaire endothéliale des mammifères, comprenant la séquence de nucléotides représentée sur la figure 6.

14. Une molécule d'ADN purifiée encodant la forme mature de 115 acides aminés du facteur II de croissance vasculaire endothéliale de mammifères, comprenant la séquence de nucléotides représentée sur la figure 7.

15. Molécules d'ADN purifiées, encodant un facteur II de croissance vasculaire endothéliale de mammifères, qui comprend :
a) une molécule d'ADN de sous-unité A, la sous-unité A étant choisie dans le groupe de molécules d'ADN constitué de : une molécule d'ADN encodant une forme mature de 188 acides aminés, ladite molécule d'ADN encodant une forme de 188 acides aminés, une forme de 164 acides aminés et une forme de 120 acides aminés, de façon que ladite forme mature de 188 acides aminés possède la séquence d'acides aminés représentée sur la figure 5 à partir de Ala 27 jusqu'à Arg 190, avec la substitution de Asn 140 en Lys 140 et l'insertion de la séquence d'acides aminés Lys-Ser-Val-Arg-Gly-Lys-Gly-Lys-Gly-Gln-Lys-Arg-Lys-Arg-Lys-Lys-Ser-Arg-Phe-Lys-Ser-Trp-Ser-Val entre Lys 140 et His 141 ; une forme mature de 164 acides aminés possède la séquence d'acides aminés représentée sur la figure 5 de Ala 27 à Arg 190 ; et une forme mature de 120 acides aminés possède la séquence d'acides aminés représentée sur la figure 5 de Ala 27 à Arg 190 ; avec la conversion de Asn 140 en Lys 140 et la délétion de la séquence d'acides aminés de His 141 à Arg 184 ; et
b) une molécule d'ADN de sous-unité B, dans laquelle ladite sous-unité B est choisie dans le groupe des molécules d'ADN constitué d'une molécule d'ADN encodant la forme mature de 135 acides aminés, représentée sur la figure 6 et une molécule d'ADN encodant la forme mature de 115 acides aminés représentée sur la figure 7.

16. Une molécule d'ADN purifiée, encodant le facteur II de croissance vasculaire endothéliale, qui est constituée d'une molécule d'ADN comportant une sous-unité A et une molécule d'ADN comportant une sous-unité B, choisie parmi les molécules d'ADN de la revendication 15.

17. Un vecteur contenant une molécule d'ADN telle que revendiquée dans l'une quelconque des revendications 13, 14 et 16.

18. Une cellule hôte transformée par un vecteur ADN de la revendication 17.

19. Un procédé pour la préparation du facteur II de croissance vasculaire endothéliale, comprenant la culture de la cellule hôte transformée de la revendication 18 sous les conditions appropriées pour l'expression du facteur II de croissance vasculaire endothéliale et la récupération du facteur II de croissance vasculaire endothéliale.

20. Le facteur II de croissance vasculaire endothéliale, réalisé par le procédé de la revendication 19.
